# EUROPEAN PATENT SPECIFICATION

(11) **EP 1 018 879 B1**
(45) Date of publication and mention of the grant of the patent: **01.08.2007**
(21) Application number: 97904887.3
(22) Date of filing: 28.01.1997
(51) Int. Cl.: C07D 239/95, C07D 233/20, C07D 233/28, C07D 459/00, C07D 311/22, C07D 335/06, C07D 295/08, A61K 31/495, A61K 31/135, A61K 31/435

(54) **NITROSATED AND NITROSYLATED ALPHA-ADRENERGIC RECEPTOR ANTAGONIST COMPOUNDS, COMPOSITIONS AND THEIR USES**
NITROSIERTE UND NITROSYLIERTE ALPHA-ADRENOREZEPTOREANTAGONISTEN, ZUBEREITUNGEN UND DEREN VERWENDUNGEN
COMPOSES D'ANTAGONISTES NITROSES OU NITROSYLES DU RECEPTEUR ALPHA-ADRENERGIQUE, LEURS COMPOSITIONS ET UTILISATIONS

(30) Priority: 02.02.1996 US 595732; 18.09.1996 US 714313
(43) Date of publication of application: 19.07.2000
(60) Divisional application: 07011814.6
(73) Proprietor: Nitromed, Inc., Boston, MA 02118 (US)
(72) Inventor: GARVEY, David, S., Dover, MA 02030 (US); SCHROEDER, Joseph, D., Cambridge, MA 02141 (US); SAENZ DE TEJADA, Inigo, E-08023 Madrid (ES)
(74) Representative: Wright, Robert Gordon McRae
(86) International application number: PCT/US1997/001294
(87) International publication number: WO 1997/027749

(56) References cited:
- WO-A-93/12068
- WO-A-95/05172
- WO-A-95/07075
- WO-A-96/32118
- GB-A- 1 249 261
- GB-A- 1 533 529
- GB-A- 1 548 856
- US-A- 3 331 855
- US-A- 3 574 212
- US-A- 3 940 387
- US-A- 3 997 666
- US-A- 4 060 615
- US-A- 4 959 360
- US-A- 5 096 916
- US-A- 5 236 904
- US-A- 5 380 758
- US-A- 5 399 581
- US-A- 5 403 847
- US-A- 5 447 912
- US-A- 5 474 535
- ZORGNIOTTI A W: "EXPERIENCE WITH BUCCAL PHENTOLAMINE MESYLATE FOR IMPOTENCE" INTERNATIONAL JOURNAL OF IMPOTENCE RESEARCH, STOCKTON, BASINGSTOKE, GB, vol. 6, no. 1, 1 March 1994 (1994-03-01), pages 37-41, XP000653737 ISSN: 0955-9930
- REGUNATHAN S ET AL: "IMIDAZOLINE RECEPTORS AND THEIR ENDOGENOUS LIGANDS" ANNUAL REVIEW OF PHARMACOLOGY AND TOXICOLOGY, ANNUAL REVIEW INC., PALO ALTO, CA, US, vol. 36, 1996, pages 511-544, XP000892884 ISSN: 0362-1642
- WELBOURN, A.P. ET AL.: "Alpha-Adrenoceptor Reagents. 4. Resolutions od Some Potent Selective Prejunctional alpha2-adrenoceptor Antagonists" J. MED. CHEM., vol. 29, 1986, pages 2000-2003, XP001069078
- URECH, E. ET AL.: "2-Aminoalkyl-imidazoline" HELVETICA CHIMICA ACTA, 1950, pages 1386-1407, XP001069616
- MITANI, K. ET AL.: "Novel Phenoxyalkylamine Derivatives. V. Synthesis, alpha-Blocking Activity and Quantitative Structure-Activity analysis of alpha-[(Phenoxyethylamino)propyl] -alpha-phenylacetonitrile Derivatives" CHEM. PHARM. BULL., vol. 36, no. 10, 1988, pages 4121-4135, XP001069068
- MELCHIORRE, C. ET AL.: "2[[[2-(2,6-Dimethoxyphenoxy)ethyl]amino]- methyl]-1,4-benzoxathian:A New antagonist with High potency and Selectivity toward alpha1-Adrenoreceptors" J. MED. CHEM., vol. 27, no. 12, 1984, pages 1535-1536, XP001069666
- DEWAR, G.H. ET AL.: "Some potential alpha-adrenoceptor blocking 1,4-benzodioxanes and 2,6-dimethoxyphenoxyethylamines" EUR. J. MED. CHEM. CHIM. THER., vol. 18, no. 3, 1983, pages 286-290, XP001069675
- PIGINI M ET AL: "Structure-Activity Relationships in 1,4-Benzodioxan-Related Compounds. Investigation on the Role of the Dehydrodioxane Ring on alpha(1)-Adrenoreceptor Blocking Activity" JOURNAL OF MEDICINAL CHEMISTRY, AMERICAN CHEMICAL SOCIETY. WASHINGTON, US, vol. 31, no. 12, 1988, pages 2300-2304, XP002123239 ISSN: 0022-2623
- CHENARD B L ET AL: "SEPARATION OF ALPHA1 ADRENERGIC AND N-METHYL-D-ASPARTATE ANTOGONISTACTIVITY IN A SERIES OF IFENPRODIL COMPOUNDS" JOURNAL OF MEDICINAL CHEMISTRY, AMERICAN CHEMICAL SOCIETY. WASHINGTON, US, vol. 34, no. 10, 1 October 1991 (1991-10-01), pages 3085-3090, XP000564617 ISSN: 0022-2623
- WETZEL J M ET AL: "DISCOVERY OF ALPHA 1A-ADRENERGIC RECEPTOR ANTAGONISTS BASED ON THE L-TYPE CA2+ CHANNEL ANTAGONIST NIGULDIPINE" JOURNAL OF MEDICINAL CHEMISTRY, AMERICAN CHEMICAL SOCIETY. WASHINGTON, US, vol. 38, no. 10, 12 May 1995 (1995-05-12), pages 1579-1581, XP000941535 ISSN: 0022-2623
- ANDERSSON, K.E. ET AL.: "Oral alpha adrenoceptor blockade as a treatment of erectile dysfunction" WORLD J UROL, vol. 19, 2001, pages 9-13, XP002198450
- TAM, S. W. ET AL.: "Yohimbine: a clinical review" PHARMACOLOGY & THERAPEUTICS, vol. 91, 2001, pages 215-243, XP002198451
- ANDERSSON, K.E.: "Pharmacology of Penile Erection" PHARMACOLOGICAL REVIEWS, vol. 53, no. 3, 2001, pages 417-450, XP001070925

## Description

This invention generally relates to α-adrenergic receptor antagonists, compositions containing them and their use in treating human male impotence.

Erectile dysfunction or impotence is a widespread disorder that is thought to affect about ten to fifeteen percent of adult men. Some pharmacological methods of treatment are available. Such methods, however, have not proven to be highly satisfactory or without potentially severe side-effects. Papaverine is now widely used to treat impotence. although papaverine is ineffective in overcoming impotence due, at least in part. to severe atherosclerosis. Papaverine is effective in cases where the dysfunction is psychogenic or neurogenic and severe atherosclerosis is not involved. Injection of papaverine, a smooth muscle relaxant, or phenoxybenzamine, a non-specific antagonist and hypotensive, into a corpus cavernosum has been found to cause an erection sufficient for vaginal penetration however. these treatments are not without the serious and often painful side effect of priapisim. Also, in cases where severe atherosclerosis is not a cause of the dysfunction, intracavemosal injection of phentolamine. an α-adrenergic antagonist, is used. As an alternative or, in some cases, an adjunct to α-adrenergic blockade, prostaglandin E1 (PGE1) has been administered via intracavernosal injection. A major side effect frequently associated with intracorprally delivered PGEI is penile pain and burning. Thus, there is a need for treatments of human male impotence without the undesirable side effects of those agents currently used.

Nitric oxide (NO) and NO donors have been recognized as mediators of nonvascular smooth muscle relaxation. This effect includes the dilation of the corpus cavernosum smooth muscle, an event involved in the penile erection process. However, the effects of such compounds together with a-adrenergic receptor antagonists or the modifications of α-adrenergic receptor antagonists to be directly or indirectly linked with a nitric oxide adduct have not been investigated.

In the process of arriving at the present invention it was recognized that the risk of toxicities and adverse effects that are associated with high doses of a-adrenergic receptor antagonists can be avoided by the use of such α-adrenergic receptor antagonists when nitrosated or nitrosylated or when administered in conjunction with compounds that donate, release or transfer nitric oxide. Such toxicities and adverse effects include postural hypotension. reflex tachycardia and other arrythmias, syncope and, with respect to the ergot alkaloids. nausea and vomiting and, upon prolonged or excessive administration, vascular insufficiency and gangrene of the extremities. The α-adrenergic receptor antagonists and compounds that donate, release or transfer nitric oxide work together to permit the same efficacy with lower doses of the α-adrenergic receptor antagonists.

Accordingly in one aspect the invention provides a nitrosated and/or nitrosylated α-adrenergic receptor antagonist wherein the α-adrenergic receptor antagonist is a compound that has been nitrosated and/or nitrosylated through an oxygen atom, a nitrogen atom or a sulfur atom which is a compound of formula III, or formula VIII:
wherein the compound of formula III is: 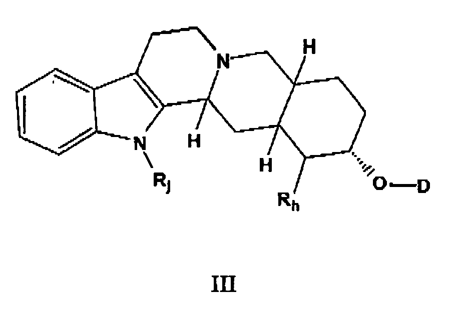 wherein Rₕ is hydrogen, -C(O)-O R_{d} or -C(O)-X;

X is (1)-Y-(C(Rₑ)(R_{f}))ₚ-G-((C(Rₑ)(R_{f}))ₚ-T-Q; or

(2) 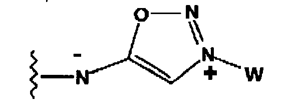 wherein Gᵢ is a covalent bond, -T-C-(O)-, -C(O)-T, -C(Y-C(O)-Rₘ)-; Rₘ is heteroaryl or heterocyclic ring; W is a heterocyclic ring or NRᵢ R'ᵢ where in Rᵢ and R'ᵢ are each independently lower alkyl, aryl or alkenyl; and wherein Rⱼ is -D or -(O)C-R_{d};
   wherein a is an integer of 2 or 3:

D is (i) -NO;
(ii) -NO₂;
(iii) -C(R_{d})-O-C(O)-Y-Z-(C(Rₑ)(R_{f})ₚ-T-Q;
(iv) -C(O)-T¹-Z-(C(Rₑ)(R_{f}))ₚ-T²-Q; or
(v) -C(O)-T(C(R_{y})(R_{z}))ₚ;
wherein R₄ is hydrogen, lower alkyl, cycloalkyl, aryl, alkylaryl or heteroaryl; Y is oxygen, sulfur, or NRᵢ; Rᵢ is hydrogen or lower alkyl; Rₑ and R_{f} are each independently hydrogen, lower alkyl, cycloalkyl, aryl, heteroaryl, arylalkyl, amino, alkylamino, amido, alkylamido, diakylamino, or carboxy, or R_{c} and R_{f} taken together are carbonyl, cycloalkyl or bridged cycloalkyl; p is an integer from 1 to 6; T is oxygen, sulfur or nitrogen; Z is a covalent bond, alkyl, cycloalkyl, aryl, heteroaryl, arylalkyl or arylheterocyclic ring; Q is -NO or -NO₂; T¹ and T² are each independently T; R_{y} and R_{z} are each independently -T¹-(C(R_{c})(R_{f}))ₚ-G-(C(Rₑ)(R_{f}))ₚ-T²-Q; G is a covalent bond, -T-C(O)-, -C(O)-T, or Y;
and wherein the compound of formula VIII is: 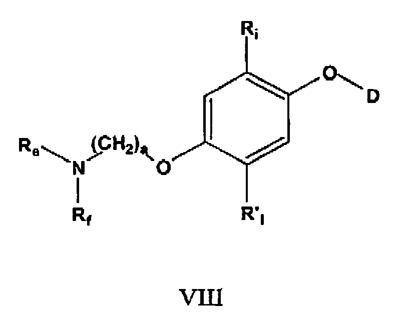 wherein a, Rᵢ, Rⱼ, Rₑ, R_{f} and D are defined as above.
According to another aspect the invention provides a composition comprising the nitrosated and/or nitrosylated α-adrenergic receptor antagonist as hereinbefore defined and a pharmaceutically acceptable carrier.
According to another aspect, the invention provides a composition comprising the nitrosated and/or nitrosylated α-adrenergic receptor antagonist of any of claims 1-3, and at least one compound that donates, transfers or releases nitric oxide, elevates endogenous synthesis levels of nitric oxide or stimulates endogenous nitric oxide synthesis, which is L-arginine, an S-nitrosothiol or
(i) a compound comprising at least one ON-O-, ON-N- or ON-C- group;
(ii) a compound comprising at least one O₂N-O-, O₂N-N-, O₂N-S or O₂N-C- group;
(iii) a N-oxo-N-nitrosoamine comprising an R₁R₂-N(O-M⁺)-NO group,
   wherein R₁ and R₂ are each independently a polypeptide, an amino acid, a sugar, a modified or unmodified oligonucleotide, a straight or branched, saturated or unsaturated, substituted or unsubstituted, aliphatic or aromatic hydrocarbon; or a heterocyclic compound; and M⁺ is a metal cation; or
(iv) a thionitrate having the structure R₁₀-S-NO₂ wherein R₁₀ is a polypeptide, an amino acid, a sugar, a modified or unmodified oligonucleotide, a straight or branched, saturated or unsaturated, substituted or unsubstituted, aliphatic or aromatic hydrocarbon; or a heterocyclic compound.

In another aspect the invention provides the use of a composition as herein described in the manufacture of a medicament for the treating human impotence in an individual in need thereof.

In another aspect the invention provides the use of the nitrosated and/or nitrosylated α-adrenergic receptor antagonist of as hereinbefore described and a compound that donates, transfers or releases nitric oxide, elevates endogenous synthesis levels of nitric oxide or stimulates endogenous nitric oxide synthesis as hereinbefore described in the manufacture of a medicament for the treating human impotence in an individual in need thereof.

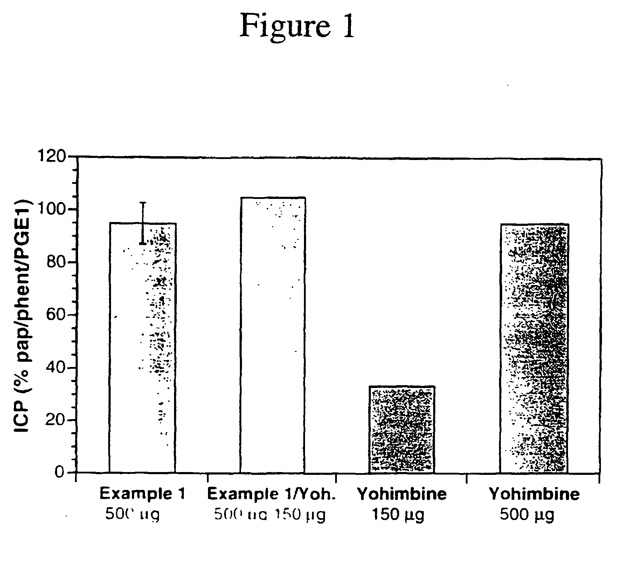

Figure 1 shows the percent peak erectile response *in vivo* compared to that produced by 150 µl of papaverine/phent amine/PGE1 (pap/phent/PGE1) (30 mg/ml: 1 mg/ml: 10 µg/ml) in the anesthetized rabbit following the intracavernosal injection of 150 µl of yohimbine (150 µg, 500 µg). Example 1 (500 µg), and a combination of yohimbine (150 µg) and Example 1 (500 µg). The ordinate is the percent response of intracavernosal pressure relative to that produced by pap/phent/PGE1 and the abscissa indicates the various drugs given.

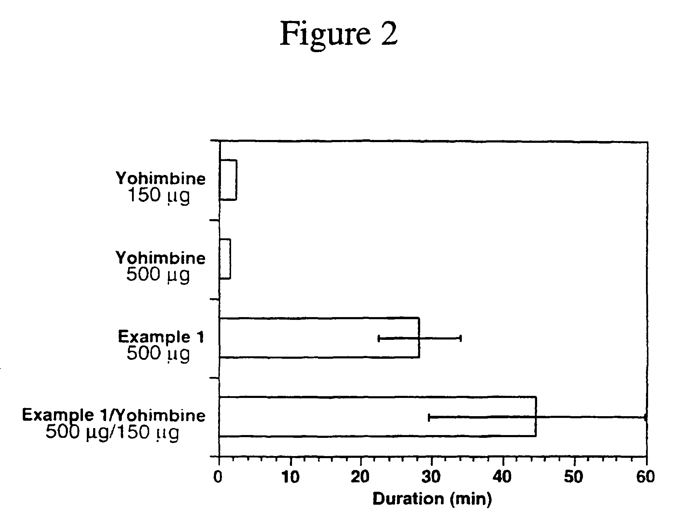

Figure 2 shows the duration of the erectile response *in vivo* in the anesthetized rabbit upon intracavernosal administration of yohimbine (150 µg, 500 µg). Example 1 (500 µg), and a combination of yohimbine (150 µg) and Example 1 (500 µg). The ordinate indicates the various drugs given and the abscissa is the duration in minutes.

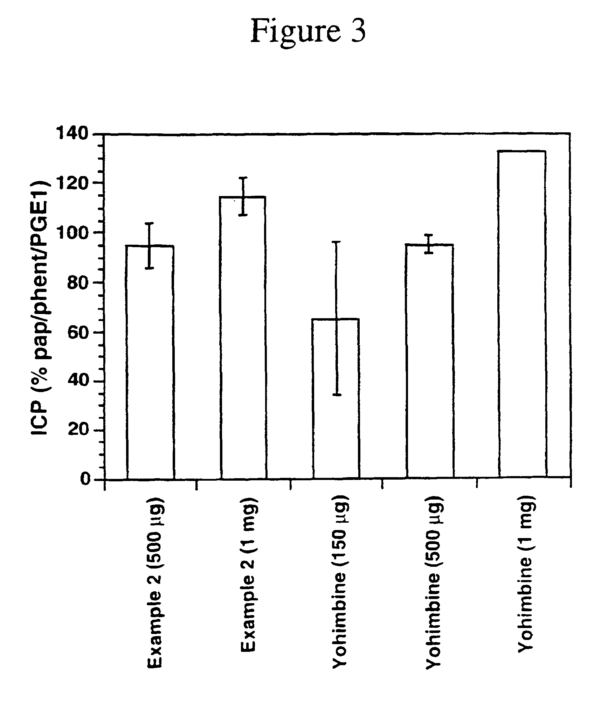

Figure 3 shows the percent peak erectile response *in vivo* compared to that produced by 150 µl of pap/phent/PGE1 (30 mg/ml: 1 mg/ml: 10 µg/ml) in the anesthetized rabbit following the intracavernosal injection of yohimbine (150 µg. 500 µg and 1 mg) and Example 2 (500 µg, 1 mg). The ordinate is the percent response of intracavernosal pressure relative to that produced by pap/phent/PGE and the abscissa indicates the various doses of yohimbine and Example 2 given.

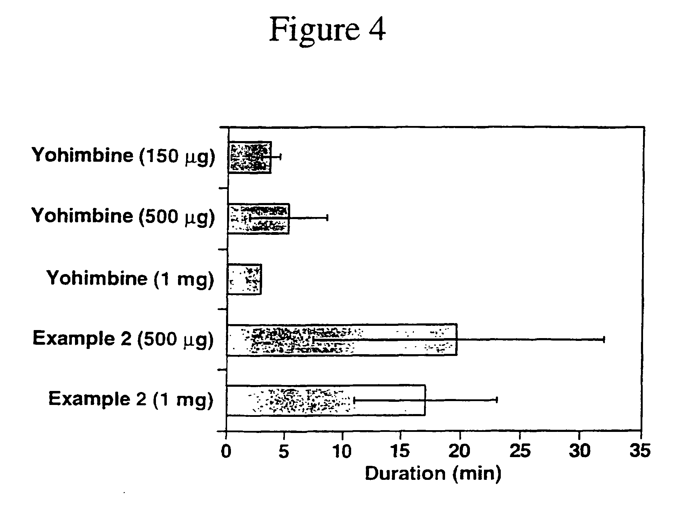

Figure 4 shows the duration of the erectile response *in vivo* in the anesthetized rabbit upon intracavernosal administration of yohimbine (150 µg, 500 µg and 1 mg) and Example 2 (500 µg and 1 mg). The ordinate indicates the various doses of yohimbine and Example 2 given and the abscissa is the duration in minutes.

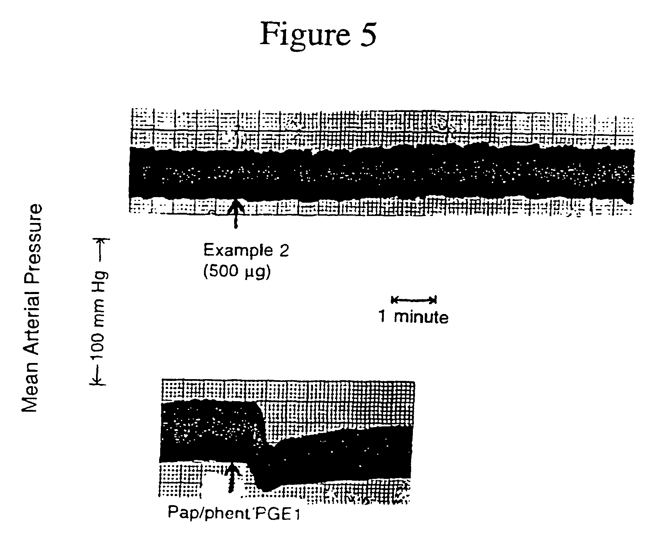

Figure 5 compares the effects of intracavernosal injections of Example 2 (500 µg) and the standard mixture of pap/phent/PGE1 on systemic blood pressure in the anesthetized rabbit.

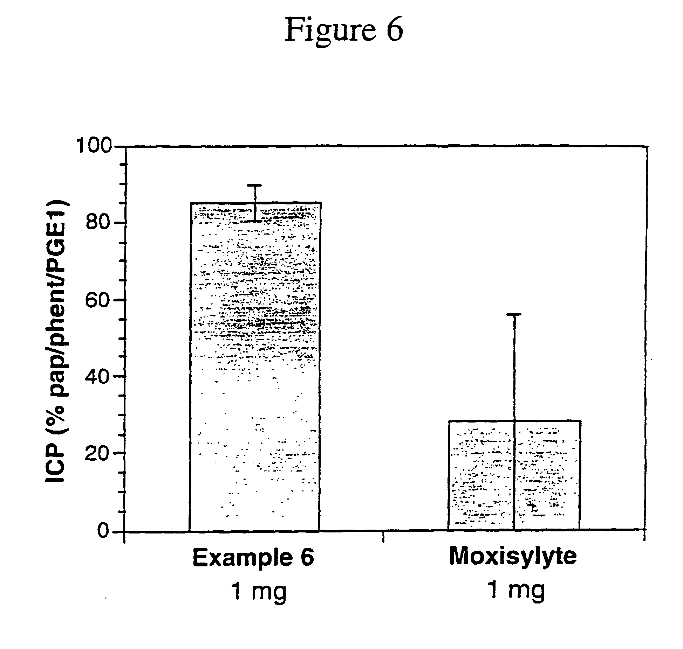

Figure 6 shows the percent peak erectile response *in vivo* compared to that produced by 150 µl of pap/phent/PGE1 (30 mg/ml: 1 mg/ml: 10 µg/ml) in the anesthetized rabbit following the intracavernosal injection of moxisylyte (1 mg) and Example 6 (1 mg). The ordinate is the percent response of intracavernosal pressure relative to that produced by pap/phent/PGE1 and the abscissa indicates the dose of moxisylyte and Example 6 given.

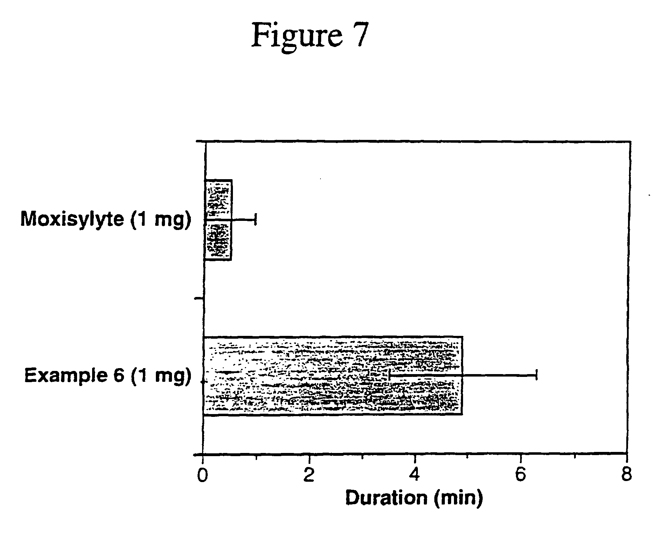

Figure 7 shows the duration of the erectile response *in vivo* in the anesthetized rabbit upon intracavernosal administration of moxisylyte (1 mg) and Example 6 (1 mg). The ordinate indicates the dose of moxisylyte and Example 6 the abscissa is the duration in minutes.

The term "lower alkyl" as used herein refers to branched or straight chain alkyl groups comprising one to ten carbon atoms, including methyl, ethyl propyl, isopropyl, n-butyl, t-butyl, neopentyl and the like.

The term "alkoxy" as used herein refers to R₅₀O- wherein R₅₀ is lower alkyl as defined above. Representative examples of alkoxy groups include methoxy, ethoxy, t-butoxy and the like.

The term "alkoxyalkyl" as used herein refers to an alkoxy group as previously defined appended to an alkyl group as previously defined. Examples of alkoxyalkyl include, but are not limited to, methoxymethyl, methoxyethyl, isopropoxymethyl and the like.

The term "hydroxy" as used herein refers to -OH.

The term "hydroxyalkyl" as used herein refers to a hydroxy group as previously defined appended to a alkyl group as previously defined.

The term "alkenyl" as used herein refers to a branched or straight chain C₂-C₁₀ hydrocarbon which also comprises one or more carbon-carbon double bonds.

The term "amino" as used herein refers to -NH₂.

The term "carboxy" as used herein refers to -C(O)O-.

The term "nitrate" as used herein refers to -O-NO₂.

The term "amido" as used herein refers to -C(O)NH-.

The term "alkylamino" as used herein refers to R₁₁NH- wherein R₁₁ is a lower alkyl group, for example, methylamino, ethylamino, butylamino, and the like.

The term "alkylamido" as used herein refers to -C(O)NR₁₁- wherein R₁₁ is defined as above.

The term "dialkylamino" as used herein refers to R₁₂R₁₃N- wherein R₁₂ and R₁₃ are independently selected from lower alkyl, for example dimethylamino, diethylamino, methyl propylamino and the like.

The term "nitro" as used herein refers to the group -NO₂ and "nitrosated" refers to compounds that have been substituted therewith.

The term "nitroso" as used herein refers to the group -NO and "nitrosylated" refers to compounds that have been substituted therewith.

The term "aryl" as used herein refers to a mono- or bicyclic carbocyclic ring system having one or two aromatic rings including, but not limited to, phenyl, naphthyl, tetrahydronaphthyl, indanyl, indenyl, and the like. Aryl groups (including bicyclic aryl groups) can be unsubstituted or substituted with one, two or three substituents independently selected from loweralkyl, haloalkyl, alkoxy, amino, alkylamino, dialkylamino, hydroxy, halo, and nitro. In addition, substituted aryl groups include tetrafluorophenyl and pentafluorophenyl.

The term "arylalkyl" as used herein refers to a lower alkyl radical to which is appended an aryl group. Representative arylalkyl groups include benzyl, phenylethyl, hydroxybenzyl, fluorobenzyl fluorophenylethyl and the like.

The term "cycloalkyl" as used herein refers to an alicyclic group comprising from 3 to 7 carbon atoms including, but not limited to, cyclopropyl, cyclobutyl, cyclopentyl, cyclohexyl and the like.

The term " halogen" or "halo" as used herein refers to I, Br, Cl, or F. The term "haloalkyl" as used herein refers to a lower alkyl radical, as defined above, bearing at least one halogen substituent, for example, chloromethyl, fluoroethyl or trifluoromethyl and the like.

The term "heteroaryl" as used herein refers to a mono- or bi- cyclic ring system containing one or two aromatic rings and containing at least one nitrogen, oxygen, or sulfur atom in an aromatic ring. Heteroaryl groups (including bicyclic heteroaryl groups) can be unsubstituted or substituted with one, two or three substituents independently selected from lower alkyl, haloalkyl, alkoxy, amino, alkylamino, dialkylamino, hydroxy, halo and nitro. Examples of heteroaryl groups include but are not limited to pyridine, pyrazine, pyrimidine. pyridazine. pyrazole. triazole. thiazole, isothiazole, benzothiazole. benzoxazole. thiadiazole, oxazole. pyrrole. imidazole and isoxazole.

The term "heterocyclic ring" refers to any 3-, 4-, 5-, 6-. or 7-membered nonaromatic ring containing at least one nitrogen atom, oxygen atom, or sulfur atom.

The term "arylheterocyclic ring" as used herein refers to a bi- or tricyclic ring comprised of an aryl ring as previously defined appended via two adjacent carbons of the aryl group to a heterocyclic ring as previously defined.

The term "heterocyclic compounds" herein refers to mono and polycyclic compounds containing at least one heteroaryl or heterocyclic ring.

Compounds of the invention which have one or more asymmetric carbon atoms may exist as the optically pure enantiomers, pure diastereomers, mixtures of enantiomers, mixtures of diastereomers. racemic mixtures of enantiomers, diastereomeric racemates or mixtures of diastereomeric racemates. It is to be understood that the present invention anticipates and includes within its scope all such isomers and mixtures thereof.

Some of the nitrosated and nitrosylated α-antagonists of the present invention may be synthesized as shown in reaction SchemesI, VII, VIII, XI, XIX, XX and XXI presented below in which Rₐ, R_{b}, R_{c}, R_{d}, Rₑ, R_{f}, R_{g}, Rₕ, Rᵢ, R'ᵢ, Rⱼ, Rₖ, Rₗ, Rₘ, Rₙ, Rₒ, Rₚ, A₁, A₂, a, n, W and X are as defined above or as depicted in the reaction schemes for formulas III. or VIII. P¹ is an oxygen protecting group and P² is a sulfur protecting group. The reactions are performed in solvents appropriate to the reagents and materials employed are suitable for the transformations being effected. It is understood by those skilled in the art of organic synthesis that the functionality present in the molecule must be consistent with the chemical transformation proposed. This will on occasion, necessitate judgment by the routineer as to the order of synthetic steps, protecting groups required, and deprotection conditions. Substituents on the starting materials may be incompatible with some of the reaction conditions required in some of the methods described, but alternative methods and substituents compatible with the reaction conditions will be readily apparent to skilled practitioners in the art. The use of sulfur and oxygen protecting groups is well known in the art for protecting thiol and alcohol groups against undesirable reactions during a synthetic procedure and many such protecting groups are known, *c.f.,* T.H. Greene and P.G.M. Wuts, Protective Groups in Organic Synthesis, John Wiley & Sons. New York (1991).

The chemical reactions described above are generally disclosed in terms of their broadest application to the preparation of the compounds of this invention. Occasionally, the reactions may not be applicable as described to each compound included within the disclosed scope- The compounds for which this occurs will be readily recognized by those skilled in the art. In all such cases, either the reactions can be successfully performed by conventional modifications known to those skilled in the art. *e.g..* by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Nitroso compounds of formula **(III)** wherein Rₑ, R_{f}, Rₕ, Rⱼ, and p are defined as above and an O-nitrosylated ester is representative of the D group as defined above may be prepared according to Scheme VII. The alcohol group of formula 7 is converted to the ester of formula 8 wherein p. Rₑ and R_{f} are defined as above by reaction with an appropriate protected alcohol containing activated acylating agent wherein P¹ is as defined above. Preferred methods for the formation of esters are reacting the alcohol with the preformed acid chloride or symmetrical anhydride of the protected alcohol containing acid. Preferred protecting groups for the alcohol moiety are silyl ethers such as a trimethylsilyl or a tert-butyldimethylsilyl ether. Deprotection of the hydroxyl moiety (fluoride ion is the preferred method for removing silyl ether protecting groups) followed by reaction a suitable nitrosylating agent such as thionyl chloride nitrite, thionyl dinitrite, or nitrosium tetrafluoroborate in a suitable anhydrous solvent such as dichloromethane. THF, DMF. or acetonitrile with or without an amine base such as pyridine or triethylamine affords the compound of the formula IIIA.

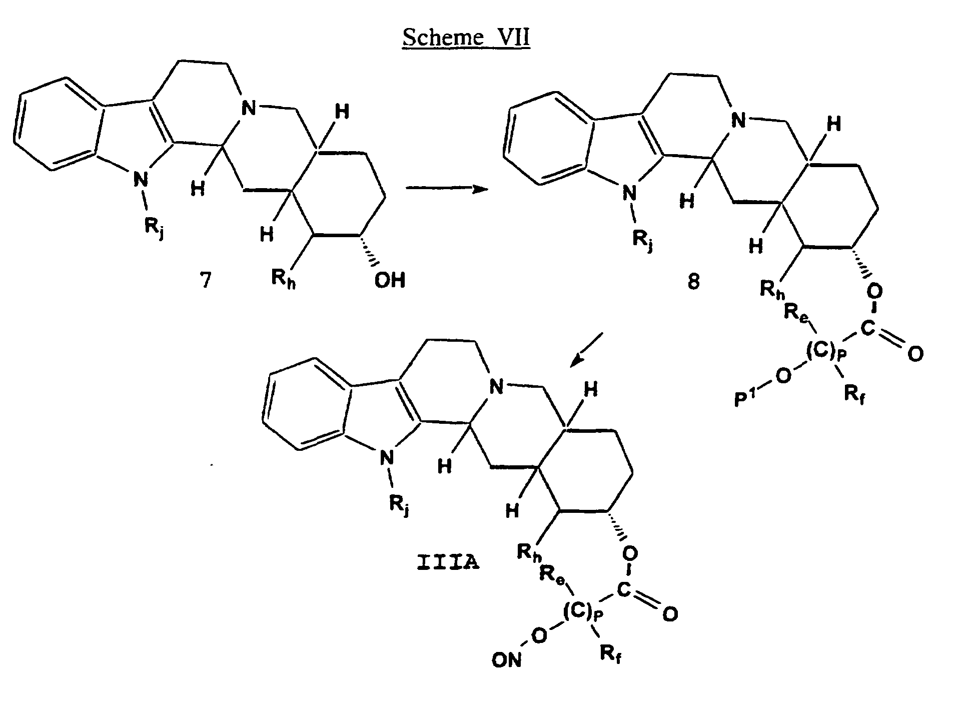

Nitroso compounds of formula (**III**) wherein Rₑ, R_{f}, Rₕ, Rⱼ, and p are defined as above and an S-nitrosylated ester is representative of the D group as defined above may be prepared according to Scheme VIII. The alcohol group of the formula 7 is converted to the ester of the formula 9 wherein p. Rₑ and R_{f} are defined as above by reaction with an appropriate protected thiol containing activated acylating agent wherein P² is as defined above. Preferred methods for the formation of esters are reacting the alcohol with the preformed acid chloride or symmetrical anhydride of the protected thiol containing acid. Preferred protecting groups for the thiol moiety are as a thioester such as a thioacetate or thiobenzoate, as a disulfide, as a thiocarbamate such as N-methoxymethyl thiocarbamate, or as a thioether such as a paramethoxybenzyl thioether. a tetrahydropyranyl thioether or a S-triphenylmethyl thioether. Deprotection of the thiol moiety (zinc in dilute aqueous acid, triphenylphosphine in water and sodium borohydride are preferred methods for reducing disulfide groups while aqueous base is typically utilized to hydrolyze thioesters and N-methoxymethyl thiocarbamates and mercuric trifluoroacetate, silver nitrate, or strong acids such as trifluoroacetic or hydrochloric acid and heat are used to remove a paramethoxybenzyl thioether, a tetrahydropyranyl thioether or a S-triphenylmethyl thioether group) followed by reaction a suitable nitrosylating agent such as thionyl chloride nitrite. thionyl dinitrite. a lower alkyl nitrite such as tert-butyl nitrite. or nitrosium tetrafluoroborate in a suitable anhydrous solvent such as methyene chloride, THF, DMF. or acetonitrile with or without an amine base such as pyridine or triethylamine affords the compound of the formula **IIIB.** Alternatively, treatment of compound 9 with a stoichiometric quantity of sodium nitrite in aqueous acid affords the compound of the formula **IIIB.**

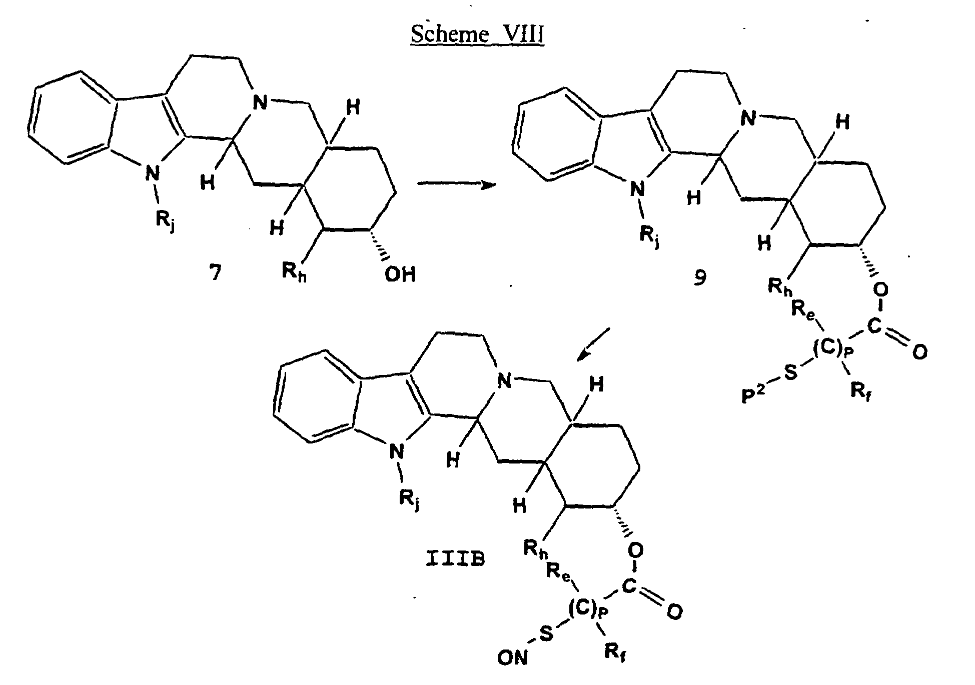

Nitro compounds of formula (**III**) wherein R_{e'} R_{f}, Rₕ, Rⱼ, and p are defined as above and an O-nitrosated ester is representative of the D group as defined above may be prepared according to Scheme IX. The alcohol group of the formula 7 is converted to the ester of the formula **IIIC** wherein p, Rₑ and Rᵣ are defined as above by reaction with an appropriate nitrate containing activated acylating agent. Preferred methods for the formation of esters are reacting the alcohol with the preformed acid chloride or symmetrical anhydride of the nitrate containing acid to afford a compound of the formula **IIIC.**

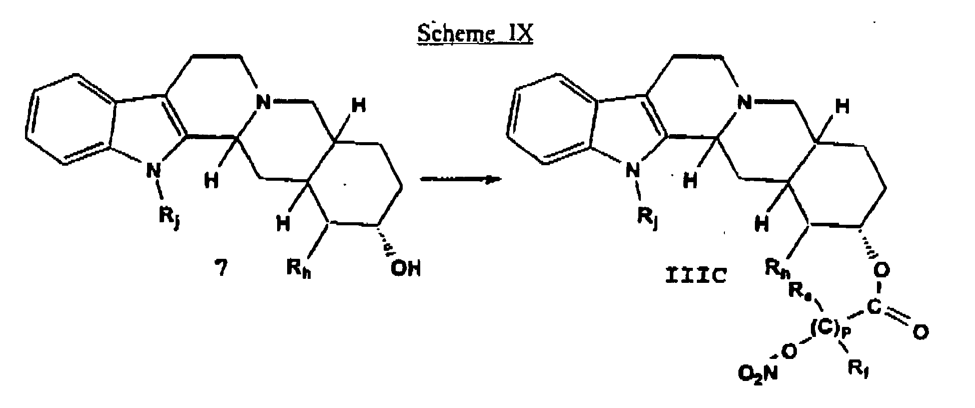

Nitroso compounds of formula (**VIII**) wherein Rₑ, R _{f}, R₁, R"ᵢ, a and p are defined as above and an O-nitrosylated ester is representative of the D group as defined above - may be prepared according to Scheme XIX. The hydroxyl group of the phenol of the formula **17** is converted to the ester of the formula **18** wherein a. p, Rₑ and R_{f} are defined as above by reaction with an appropriate protected alcohol containing activated acylating agent wherein P¹ is as defined above. Preferred methods for the formation of esters are reacting the hydroxyl with the preformed acid chloride or symmetrical anhydride of the protected alcohol containing acid. Preferred protecting groups for the alcohol moiety are silyl ethers such as a trimethylsilyl or a tert-butyldimethylsilyl ether. Deprotection of the hydroxyl moiety (fluoride ion is the preferred method for removing silyl ether protecting groups) followed by reaction a suitable nitrosylating agent such as thionyl chloride nitrite. thionyl dinitrite, or nitrosium tetrafluoroborate in a suitable anhydrous solvent such as dichloromethane, THF, DMF, or acetonitrile with or without an amine base such as pyridine or triethylamine affords the compound of the formula **VIIIA.**

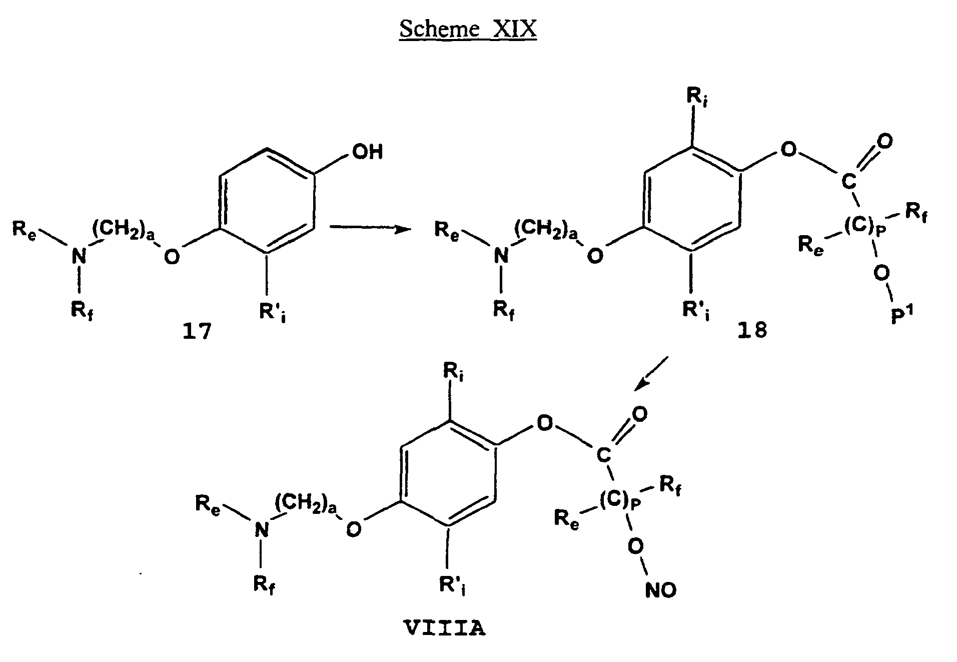

Nitroso compounds of formula **(VIII)** wherein Rₑ, R_{f}, R₁, R'ᵢ, a. and p are defined as above and an S-nitrosylated ester is representative of the D group as defined - above may be prepared according to Scheme XX. The hydroxyl group of the phenol of the formula **17** is converted to the ester of the formula **19** wherein a. p. Rₑ and R_{f} are defined as above by reaction with an appropriate protected thiol containing activated acylating agent wherein P² is as defined above. Preferred methods for the formation of esters are reacting the hydroxyl with the preformed acid chloride or symmetrical anhydride of the protected thiol containing acid. Preferred protecting groups for the thiol moiety are as a thioester such as a thioacetate or thiobenzoate, as a disulfide, as a thiocarbamate such as N- methoxymethyl thiocarbamate, or as a thioether such as a paramethoxybenzyl thioether, a tetrahydropyranyl thioether or a S-triphenylmethyl thioether. Deprotection of the thiol moiety (zinc in dilute aqueous acid, triphenylphosphine in water and sodium borohydride are preferred methods for reducing disulfide groups while aqueous base is typically utilized to hydrolyze thioesters and N-methoxymethyl thiocarbamates and mercuric trifluoroacetate. silver nitrate, or strong acids such as trifluoroacetic or hydrochloric acid and heat are used to remove a paramethoxybenzyl thioether. a tetrahydropyranyl thioether or a S-triphenylmethyl thioether group) followed by reaction a suitable nitrosylating agent such as thionyl chloride nitrite. thionyl dinitrite, a lower alkyl nitrite such as tert-butyl nitrite. or nitrosium tetrafluoroborate in a suitable anhydrous solvent such as methyene chloride. THF. DMF. or acetonitrile with or without an amine base such as pyridine or triethylamine affords the compound of the formula **VIIIB.** Alternatively, treatment of compound 17 with a stoichiometric quantity of sodium nitrite in aqueous acid affords the compound of the formula **VIIIB**.

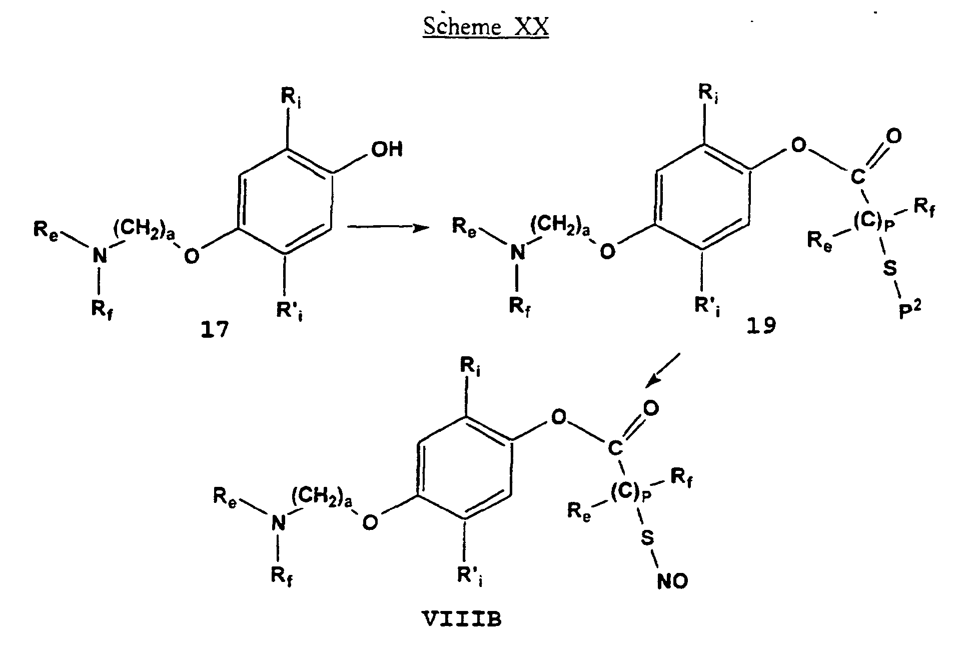

Nitro compounds of formula **(VIII)** wherein Rₑ, R_{f}, Rᵢ, R'ᵢ, a, and p are defined as above and an O-nitrosated ester is representative of the D group as defined above may be prepared according to Scheme XXI. The hydroxyl group of the phenol of the formula **15** is converted to the ester of the formula **VIIIC** wherein a. p, Rₑ and R_{f} are defined as above by reaction with an appropriate nitrate containing activated acylating agent. Preferred methods for the formation of amides are reacting the amine with the preformed acid chloride or symmetrical anhydride of the nitrate containing acid to afford the compound of the formula **VIIIC.**

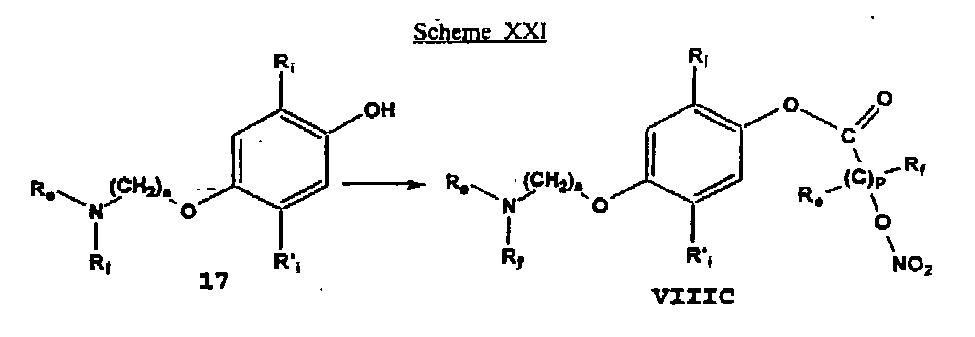

As noted above, another aspect the invention provides a composition comprising a therapeutically effective amount of an α-adrenergic receptor antagonist (α-antagonist), which can optionally be substituted with at least one NO or NO₂ moiety, and a compound that donates, transfers or releases nitric oxide as a charged species. *i.e.,* nitrosonium (NO⁻) or nitroxyl (NO⁻), or as the neutral species, nitric oxide (NO•).

The compounds that donate, transfer or release nitric oxide include those mentioned and/or exemplified below.

Nitrogen monoxide can exist in three forms: NO⁻ (nitroxyl), NO• (nitric oxide) and NO⁻ (nitrosonium). NO• is a highly reactive short-lived species that is potentially toxic to cells. This is critical. because the pharmacological efficacy of NO depends upon the form in which it is delivered. In contrast to NO•, nitrosonium and nitroxyl do not react with O₂ or O₂⁻ species, and are also resistant to decomposition in the presence of redox metals. Consequently. administration of NO equivalents does not result in the generation of toxic by-products or the elimination of the active NO moiety.

Compounds contemplated for use in the invention are nitric oxide and compounds that release nitric oxide or otherwise directly or indirectly deliver or transfer nitric oxide to a site of its activity, such as on a cell membrane*. in vivo.* As used here. the term "nitric oxide" encompasses uncharged nitric oxide (NO•) and charged nitric oxide species. particularly including nitrosonium ion (NO⁻) and nitroxyl ion (NO⁻). The reactive form of nitric oxide can be provided by gaseous nitric oxide. The nitric oxide releasing. delivering or transferring compounds, having the structure F-NO wherein F is a nitric oxide releasing, delivering or transferring moiety, include any and all such compounds which provide nitric oxide to its intended site of action in a form active for their intended purpose. As used here, the term "NO adducts" encompasses any of such nitric oxide releasing, delivering or transferring compounds. including, for example, S-nitrosothiols. S-nitrothiols. O-nitrosoalcohols, O-nitroalcohols, sydnonimines, 2-hydroxy-2-nitrosohydrazines (NONOates), (E)-alkyl-2-[(E)-hydroxyimino]-5-nitro-3-hexene amines or amides, nitrosoamines. as well a subtstates for the endogenous enzymes which synthesize nitric oxide. It is contemplated that any or all of these "NO adducts" can be mono- or poly-nitrosylated or nitrosated at a variety of naturally susceptible or artificially provided binding sites for nitric oxide or derivatives which donate or release NO.

One group of such NO adducts is the S-nitrosothiols, which are compounds that include at least one -S-NO group. Such compounds include S-nitroso-polypeptides (the term "polypeptide" includes proteins and also polyamino acids that do not possess an ascertained biological function. and derivatives thereof); S-nitrosylated amino acids (including natural and synthetic amino acids and their stereoisomers and racemic mixtures and derivatives thereof): S-nitrosylated sugars. S-nitrosylated-modified and unmodified oligonucleotides (preferably of at least 5, and more particularly 5-200. nucleotides); and an S-nitrosylated hydrocarbons where the hydrocarbon can be a branched or unbranched. and saturated or unsaturated aliphatic hydrocarbon, or an aromatic hydrocarbon; S- nitrosylated hydrocarbons having one or more substituent groups in addition to the S- nitroso group; and heterocyclic compounds. S-nitrosothiols and the methods for preparing them are described in U.S. Patent No. 5.380.758; Oae et al.. Org. Prep. Proc. Int.. 15(3):165-198 (1983); Loscalzo et al., J. Pharmacol. Exp. Ther., 249(3):726729 (1989) and Kowaluk et al., J. Pharmacol. Exp. Ther., 256:1256-1264 (1990), all of which are incorporated in their entirety by reference.

One particularly preferred embodiment of this aspect relates to S-nitroso amino acids where the nitroso group is linked to a sulfur group of a sulfur-containing amino acid or derivative thereof. For example, such compounds include the following: S-nitroso-N-acetylcysteine, S-nitroso-captopril, S-nitroso-homocysteine. S-nitroso-cysteine and S-nitroso-glutathione.

Suitable S-nitrosylated proteins include thiol-containing proteins (where the NO group is attached to one or more sulfur group on an amino acid or amino acid derivative thereof) from various functional classes including enzymes, such as tissue-type plasminogen activator (TPA) and cathepsin B; transport proteins, such as lipoproteins, heme proteins such as hemoglobin and serum albumin: and biologically protective proteins, such as the immunoglobulins and the cytokines. Such nitrosylated proteins are described in PCT Publ. Applic. No. WO 93/09806, published May 27. 1993. Examples include polynitrosylated albumin where multiple thiol or other nucleophilic centers in the protein are modified.

Further examples of suitable S-nitrosothiols include those having the structures:
(i)CH₃[C(R_{c})(R_{f})]ₓSNO
   wherein x equals 2 to 20 and Rₑ and R_{f} are as defined above;
(ii)HS[C((Rₑ)(R_{f})]ₓSNO
   wherein x equals 2 to 20; and Rₑ and R_{f} are as defined above;
(iii)ONS[C(Rₑ)(R_{f})]ₓB ; and
   (iv)H₂N-(CO₂H)-(CH₂)ₓ-C(O)NH-C(CH₂SNO)-C(O)NH-CH₂-CO₂H
   wherein x equals 2 to 20: Rₑ and R_{f} are as defined above; and B is selected from the group consisting of fluoro, C₁-C₆ alkoxy, cyano, carboxamido, cycloalkyl, arylakoxy, alkylsulfinyl, arylthio, alkylamino, dialkylamino, hydroxy, carbamoyl, N-alkylcarbamoyl, N,N-dialkylcarbamoyl. amino. hydroxyl, carboxyl, hydrogen, nitro and aryl.

Nitrosothiols can be prepared by various methods of synthesis. In general, the thiol precursor is prepared first, then converted to the S-nitrosothiol derivative by nitrosation of the thiol group with NaNO₂ under acidic conditions (pH is about 2.5) to yield the S-nitroso derivative. Acids which may be used for this purpose include aqueous sulfuric, acetic and hydrochloric acids. Alternatively, the precursor thiol may be nitrosylated by treatment with an alkyl nitrite such as tert-butyl nitrite.

Another group of such NO adducts are those wherein the compounds donate, transfer or release nitric oxide and are selected from the group consisting of compounds that include at least one ON-N- or ON-C- group. The compound that includes at least one ON-N- or ON-C- group is preferably selected from the group consisting of ON-N- or ON-C-polypeptides (the term "polypeptide" includes proteins and also polyamino acids that do not possess an ascertained biological function, and derivatives thereof): ON-N- or ON-C-amino acids(including natural and synthetic amino acids and their stereoisomers and racemic mixtures); ON-N- or ON-C-sugars; ON-N- or ON-C-modified and unmodified oligonucleotides (preferably of at least 5, and more particularly 5-200, nucleotides). ON- O-, ON-N- or ON-C-hydrocarbons which can be branched or unbranched, saturated or unsaturated aliphatic hydrocarbons or aromatic hydrocarbons: ON-N- or ON-C- hydrocarbons having one or more substituent groups in addition to the ON-N- or ON-C- group; and ON-N- or ON-C-heterocyclic compounds.

Another group of such NO adducts is the nitrites which have an -O-NO group wherein the organic template to which the nitrite group is appended is a protein, polypeptide, amino acid, carbohydrate, branched or unbranched and saturated or unsaturated alkyl, aryl or a heterocyclic compound. A preferred example is the nitrosylated form of isosorbide. Compounds in this group form S-nitrosothiol intermediates *in vivo* in the recipient human or other animal to be treated and can therefore include any structurally analogous precursor R-O-NO of the S-nitrosothiols described above.

Another group of such adducts are nitrates which donate, transfer or release nitric oxide and are selected from the group consisting of compounds that include at least one at least one O₂N-O-, O₂N-N-. O₂N-S- or O₂N-C- group. Preferred among these are those selected from the group consisting of O₂N-O-, O₂N-N-, O₂N-S- or O₂N-C- polypeptides; O₂N-O-, O₂N-N-. O₂N-S- or O₂N-C-amino acids; O₂N-O-, O₂N-N- O₂N- S- or O₂N-C-sugars; O₂N-O-. O₂N-N-, O₂N-S- or O₂N-C-modified and unmodified oligonucleotides; O₂N-O-, O₂N-N-, O₂N-S- or O₂N-C- hydrocarbons which can be branched or unbranched, saturated or unsaturated aliphatic hydrocarbons or aromatic hydrocarbons: O₂N-O-. O₂N-N-. O,N-S- or O,N-C- hydrocarbons having one or more substituent groups in addition to the O₂N-O-. O₂N-N-, O₂N-S- or O₂N-C-group ; and O₂N-O-. O₂N-N-,O₂N-S- or O₂N-C-heterocyclic compounds. Preferred examples are isosorbide dinitrate and isosorbide mononitrate.

Another group of such NO adducts is the nitroso-metal compounds which have the structure (R)ᵥ-A-M-(NO)ᵥ. R includes polypeptides (the term "polypeptide" includes proteins and also polyamino acids that do not possess an ascertained biological function, and derivatives thereof); amino acids (including natural and synthetic amino acids and their stereoisomers and racemic mixtures and derivatives thereof); sugars; modified and unmodified oligonucleotides (preferably of at least 5, and more particularly 5-200. nucleotides); and a hydrocarbon where the hydrocarbon can be a branched or unbranched, and saturated or unsaturated aliphatic hydrocarbon, or an aromatic hydrocarbon: hydrocarbons having one or more substituent groups in addition to the A-nitroso group: and heterocyclic compounds. A is S. O. or N. n and x are each integers independently selected from 1, 2 and 3, and M is a metal, preferably a transition metal. Preferred metals include iron, copper, manganese, cobalt, selenium and luthidium. Also contemplated are N-nitrosylated metal centers such as nitroprusside.

Another group of such adducts are N-oxo-N-nitrosoamines which donate, transfer or release nitric oxide and have a R₁R₂-N(O-M⁻)-NO group wherein R₁ and R₂ include polypeptides, amino acids, sugars, modified and unmodified oligonucleotides, hydrocarbons where the hydrocarbon can be a branched or unbranched, and saturated or unsaturated aliphatic hydrocarbon or an aromatic hydrocarbon, hydrocarbons having one or more substituent groups and heterocyclic compounds. M⁻ is a metal cation, such as, for example, a Group I metal cation.

Another group of such adducts are thionitrates which donate, transfer or release nitric oxide and have the structure R₁₀-S-NO₂ wherein R₁₀ is as described above for the N-oxo-N-nitrosoamines.

Agents which stimulate endogenous NO synthesis such as L-arginine, the substrate for nitric oxide synthase are also suitable for use in accordance with the invention.

When administered *in vivo,* the nitric oxide may be administered in combination with pharmaceutical carriers and in dosages described herein.

In another aspect the invention provides a method of treating male impotence in an individual in need thereof by administering to the individual a therapeutically effective amount of a composition comprising a nitrosated or nitrosylated α-antagonist of the invention in a pharmaceutically acceptable carrier.

In another aspect the invention provides a method of treating male impotence in an individual in need thereof which comprises treating an individual for male impotence by administering to the individual a therapeutically effective amount of a composition comprising an α-adrenergic receptor antagonist (α-antagonist), which can optionally be substituted with at least one NO or NO, moiety. and a compound that donates, transfers or releases nitric oxide in a pharmaceutically acceptable carrier.

Total daily dose administered to a host in single or divided doses may be in amounts, for example, from about 1 to about 100 mg/kg body weight daily and more usually about 3 to 30 mg/kg. Dosage unit compositions may contain such amounts of submultiples thereof to make up the daily dose.

The amount of active ingredient that may be combined with the carrier materials to produce a single dosage form will vary depending upon the host treated and the particular mode of administration.

The dosage regimen for treating a disease condition with the compounds and/or compositions of this invention is selected in accordance with a variety of factors, including the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized and whether the compound is administered as part of a drug combination. Thus, the dosage regimen actually employed may vary widely and therefore may deviate from the preferred dosage regimen set forth above.

The compounds of the present invention may be administered orally, parenterally or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrastemal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1. 3-butanediol. Among the acceptable vehicles and solvents that may be employed are water. Ringer's solution, and isotonic sodium chloride solution. In addition, sterile, fixed oils are conventionally employed an a solvent or suspending medium. For this purpose any bland fixed oil may be employed including synthetic mono- or diglycerides, in addition, fatty acids such as oleic acid find use in the preparation of injectables.

Solid dosage forms for oral administration may include capsules, tablets, pills, powders, granules and gels. In such solid dosage forms, the active compound may be admixed with at least one inert diluent such as sucrose lactose or starch. Such dosage forms may also comprise, as in normal practice, additional substances other than inert diluents, e.g.. lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more compounds which are known to be effective against the specific disease state that one is targeting for treatment. The compositions of the invention can be administered as a mixture of an α-antagonist and a nitric oxide donor, they can also be used in combination with one or more compounds which are known to be effective against the specific disease state that one is targeting for treatment.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration.

### Example 1

### N-(N-L-g-glutamyl- S-Nitroso-L-cysteinyl)glycine

N-(N-L-g-glutamyl-L-cysteinyl)glycine (100 g, 0.325 mol) was dissolved in deoxygenated water (200 ml) and 2N HCl (162 ml) at room temperature and then the reaction mixture was cooled to 0°C. With rapid stirring, a solution of sodium nitrite (24.4 g, 0.35 mol) in water (40 ml) was added. Stirring with cooling of the reaction mixture was continued for approximately 1 hour after which time the pink precipitate which formed was collected by vacuum filtration. The filter cake was resuspended in chilled 40% acetone-water (600 ml) and collected by vacuum filtration. The filter cake was washed with acetone (2 X 200 ml) and ether (100 ml) and then dried under high vacuum at room temperature in the dark to afford the title compound as a pink powder. ¹H NMR (D₂O) : 1.98 (m, 2H). 2.32 (t. 2H), 3.67 (t, 1H), 3.82 (s 2H). 3.86 (dd. 1H). 3.98 (dd. 1H). 4.53 (m. 1H).

### Example 2

### 2-Acyl-17α(3-methyl-3-nitrosothiolbutoxy)vohimban-16α-carboxylic acid methyl ester hydrochloride salt

### 2a. 3-Methyl-3(2-tetrahydropyranyl)thiobutyric acid

3-Methyl-3-thiobutyric acid (4.2 g, 31 mmol), dihydropyran (2.8 ml, 31 mmol), and 200 µl of 4 N HCl/Et₂O were allowed to stand at room temperature overnight. The volatiles were evaporated *in vacuo* (2 mm Hg) yielding 6.6 g (30 mmol) of material which was used without further purification. ¹H-NMR (CDCl₃): 4.92 (d. J = 8.1 Hz, 1H), 4.09 (d. J = 10.5 Hz, 1H), 3.49-3.56 (mult. 1H), 2.73 (dd, J = 1.2 and 13.7 Hz, 1H). 2.64 (d. J = 13.8 Hz, 1H). 1.84-1.89 (mult 2H). 1.55-1.69 (mult. 4H). 1.51(s, 3H), 1.42 (s. 3H).

### 2b. 3,3'-Dimethyl-3,3'(2-ditetrahydropyranyl)thiobutyric acid anhydride

The product of Example 2a (1.1 g. 5 mmol) and triethylamine (710 µl, 5 mmol) was dissolved in ethyl acetate (50 ml) and cooled to 0° C. Triphosgene (250 mg, 0.85 mmol) was added all in one portion and the reaction was stirred at 0° C for 15 minutes then warmed to room temperature with continued stirring for 30 minutes. The precipitate which formed was removed by filtration and the filtrate was concentrated by rotary evaporation to afford 1.0 g (5 mmol) of the title compound. ¹H-NMR (CDCl₃): 5.03-5.06 (mult. 2H). 4.04-4.08 (mult, 2H), 3.46-3.51 (mult, 2H), 2.89 (d. J = 15.7 Hz, 2H), 2.77 (d. J = 15.6 Hz. 2H), 1.79-1.88 (mult. 4H). 1.51-1.67 (mult. 8H). 1.54 (s. 6H). 1.49 (s, 6H).

### 2c. 17a (3-methyl-3-tetrahydropyranylthiolbutoxy)vohimban-16α-carboxylic acid methyl ester

To a solution of yohimbine (1.6 g, 4.5 mmol) in pyridine (6 ml) was added the product of Example 2b (2.5 g. 6 mmol) and 4-dimethylaminopyridine (730 mg, 6 mmol). The reaction mixture was stirred at room temperature for 6 days. Acetonitrile (50 ml) was added to the reaction and then all of the volatile components were evaporated *in vacuo.* The residue was dissolved in ethyl acetate (100 ml) and washed with a 10 % solution of aqueous sodium carbonate. The aqueous wash was then back extracted once with ethyl acetate. The combined organic extracts were washed with H₂O, brine, and then dried over anhydrous sodium sulfate. Treatment of the solution with activated charcoal followed by filtration and concentration of the filtrate *in vacuo* gave 2.8 g of a dark syrup.

Chromatography on silica gel eluting with 1:1 hexane/ethyl acetate containing 1 % by volume triethylamine afforded 670 mg (20%) of the title compound. ¹H-NMR (CDCl₃): 7.76 (s, 1H), 7.46 (d. J = 7.2 Hz, 1H), 7.29 (dd, J = 1.0 and 7.0 Hz, 1H), 7.12 (ddd; J = 1.3, 7.1. and 7.1 Hz; 1H), 7.07 (ddd; J = 1.1, 7.2, and 7.2 Hz; 1H). 5.46 (d, J = 2.6 Hz, 1H), 5.07-5.11 (mult, 1H). 4.06-4.11 (mult, 1H), 3.69 (s, 3H). 3.47-3.55 (mult, 1H). 3.39 (d, J = 10.4 Hz, 1H). 3.02-3.12 (mult, 2H), 2.97 (dd, J = 4.5 and 12.2 Hz, 1H), 2.80 (d. J = 14.3 Hz, 1H). 2.71 (mult, 1H). 2.69 (d, J = 13.2 Hz. 1H). 2.61-2.65 (mult. 1H). 2.39 (dd. J = 2.6 and 11.6 Hz, 1H). 2.23-2.33 (mult, 2H), 1.71-2.07 (mult, 5H), 1.58-1.69 (mult. 8H). 1.51 (s. 3H). 1.49 (s. 3H). Anal Calcd for (C₃₁H₄₂N₂O5S-1/2 H₂O): C. 66.05; H. 7.69: N. 4.97: S. 5.69. Found C. 65.74; H, 7.33; N. 4.88: S, 5.57.

### 2d. 2-Acyl- 17a(3 -methyl-3 -thiolbutoxy)yohimban-16α-carboxylic acid methyl ester

The product of Example 2c (620 mg, 1.1 mmol) was refluxed in a mixture of acetic acid (5 ml) and acetyl chloride (5 ml) for 4 hours. The solvent was evaporated *in vacuo* (2 mm Hg). The residue was partitioned between 5% aqueous ammonium hydroxide and ethyl acetate. The aqueous wash was extracted with ethyl acetate. The combined organic extracts were washed with brine and dried over anhydrous sodium sulfate. The solvent was evaporated *in vacuo* and the residue was chromatographed on silica gel eluting with 1:1 hexane/ethyl acetate containing 1% by volume triethylamine to - afford 210 mg (34 %) of 2-acyl-17α.(3-methyl-3-thioacetylbutoxy)yohimban-16 α-carboxylic acid methyl ester. This diacetate (180 mg. 0.32 mmol) was dissolved in acetic acid (4 ml) to which was added mercuric trifluoroacetate (190 mg. 0.45 mmol) and the reaction mixture was stirred at room temperature for 2 hours. The volatiles were evaporated *in vacuo* leaving a gum which was triturated with 1N HCl (6 ml) to afford a yellow powder. The powder was partitioned between ethyl acetate and 10% aqueous ammonium hydroxide. The organic phase was filtered through Celite to remove the gray solid which was present and then the filtrate was washed with brine and then dried over anhydrous sodium sulfate.

Evaporation of the volatiles *in vacuo* afforded a solid which was chromatographed on silica gel eluting with a gradient of with 1:1 hexane/ethyl acetate containing I % by volume triethylamine to ethyl acetate containing I % by volume triethylamine to yield 60 mg (37 %) of the title compound as a white powder. ¹H-NMR (CDCl₃): 7.81 (d. J = 7.0 Hz 1H). 7.41 (d. J = 6.8 Hz 1H). 7.23-7.29 (mult. 2H). 5.46 (s. 1H). 4.17 (d. J = 9.9 Hz. 1H). 3.64 (s.3H). 3.11-3.15 (mult. 1H), 3.00 (dd. J = 3.5 and 12.4 Hz 1H). 2.64-2.84 (mult. 10H). 2.31 (dd, J = 2.6 and 11.7 Hz. 1H), 2.24 (d, J = 12.7 Hz. 1H). 2.04-2.0 8 (mult. 2H). 1.41-1.62 (mult, 11H). ¹³C-NMR (CDCl₃): 171.6. 170.7. 169.5. 13 7.3. 136.4, 129.6, 124.1, 122.9. 118.3. 117.2. 114.6, 70.0, 61.0, 59.8, 51.9, 51.8, 50.9, 47.7. 45.6, 37.8, 37.6, 36.23, 36.2, 33.2, 29.9, 27.1, 23.8, 22.3.

### 2e. 2-Acyl-17a(3-methyl-3-nitrosothiolbutoxy)yohimban-16α-carboxylic acid methyl ester hydrochloride salt

To a slurry of the compound of Example 2d (40 mg. 0.078 mmol) in 1:1 methanol/1N HCl (4 ML) with dimethylformamide (400 µl) was added a solution of sodium nitrite (11 mg. 0. 16 mmol) in H₂O (200 µl). The white powder turned green as the slurry was stirred at room temperature for 25 minutes. At this juncture dimethylformamide (600 µl) and additional aqueous sodium nitrite (11 mg in 200 µl of H₂O) was added and stirring at room temperature was continued for an additional 15 minutes. The reaction mixture was partitioned between CHCl₃ and H₂O adding 10% aqueous ammonium hydroxide to the aqueous phase until basic to pH paper. The aqueous layer was extracted with CHCl₃ and the combined organic extracts were washed with brine and then dried over anhydrous sodium sulfate. The volatiles were evaporated *in vacuo* and the residue was dissolved in ether. The product was precipitated with ethereal HC 1 to afford 19 mg of the title compound as a green solid. ¹H-NMR (CDCl₃): 7.81 (dd, J = 1.7 and 6.8 Hz. 1H). 7.42 (d. J = 6.8 Hz. 1H). 7.23-7.29 (mult. 2H). 5.43 (d. J = 2.6 Hz. 1H). 4.15 (d. J = 9.8 Hz. 1H). 3.63 (s. 3H). 3.36 (d, J = 15.1 Hz. 1H). 3.30 (d. J = 15.1 Hz. 1H). 3.12 (dd, J = 4.9 and 11.0 Hz, 1H). 3.00 (dd, J = 3.7 and 12.3 Hz. 1H). 2.72 (s. 3H). 2.63 -2.82 (mult. 3H). 2.31 (dd, J = 2.6 and 11.7 Hz. 1H). 2.03 (s. 3H). 2.00 (s. 3H). 1.0-2.0 (mult, 9H).

### Example 3

### 2-{[β-(4-(3-S-Nitroso-3-methyl-butyric acid)phenyl) ethytlaminomethyl}-1-tetralone ester hydrochloride 3a. 2-{[β-(4-Hydroxyphenyl) ethyl] t-butoxycarbonylaminomethyl}-1-tetralone

2-{[β-4-Hydroxyphenyl) ethyl] aminomethyl}-1-tetralone (3.39 g. 11.5 mmol) was dissolved in dichloromethane (50 mL) and di-tert-butyldicarbonate (2.50 g. 11.5 mmol) was added. The reaction mixture was stirred for 100 minutes at room temperature. The solvent was evaporated, and the residue was purified by flash chromatography on silica-gel, eluting with hexane/ethyl acetate (3:1) to give 2.32 g (51 %) of the title compound. ¹H NMR (CDCl₃, 300 MHz) 1.44 (s, 9 H), 1.61-1.89 (m, 1 H). 2.15-2.29 (m, I H). 2.50-2.85 (m. 4 H), 2.90-3.08 (m. 2 H). 3.29-3.45 (m, 3 H), 3.49-3.64 (m, 1 H), 6.76 (d, 2 H). 7.04 (d. 2H), 7.19-7.32 (m. 2 H). 7.39-7.50 (m, 1 H), 8.01 (d. 1 H).

### 3b. 2-{[β-(4-(3-Tetrahydropyranylthio-3-methyl-butyric acid)phenyl) ethyl]aminomethyl}-1-tetralone ester

The product of Example 3a (0.300 g, 0.76 mmol) was dissolved in pyridine (0.5 mL) and a solution of the product of Example 2b (0.397 g. 0.95 mmol) in pyridine (0.5 mL) was added. The resulting solution was stirred for 18 hours at room temperature. The solvent was evaporated, and the residue was purified by flash chromatography on silica-gel, eluting with hexane/ethyl acetate (4:1) to give 0.332 g (73 %) of the title compound. ¹H NMR (CDCl₃. 300 MHz) 1.44 (s. 9 H). 1.56 (d. 6 H), 1.52-1.78 m, 6 H). 1.66-1.97 (m, 1 H). 2.16-2.31 (m. 1 H). 2.73- 3.06 (dd. overlapping with multiplet, 7H), 3.33-3.67 (m, 5 H), 4.05-4.17 (m, I H). 5.09-5.17 (m. 1 H), 7.01 (d, 2 H), 7.13-7.36 (m. 4 H), 7.47 (t. 1 H). 8.01 (d. 1 H).

### 3c. 2-{[β-(4-(3-Mercapto-3-methyl-butyric acid)phenyl) ethyl]-t-buyoxycarbonylaminomethyl}-1-tetralone ester

The product of Example 3b (0.192 g. 0.32 mmol) was dissolved in methanol (2 mL) and a solution of silver nitrate (0.117g. 0.69 mmol) in water (0.4 mL) was added. The resulting mixture was stirred for I hour at room temperature. The solvent was evaporated, the residue was suspended in acetone/water (1:10) and 1N HCl (1 mL) was added. After stirring for 18 hours at room temperature, the precipitate was filtered and filtrate was extracted with dichloromethane. The organic layer was washed with brine, dried over anhydrous sodium sulfate and concentrated in vacuo to give 0.085 g (51 %) of the title compound. ¹H NMR (CDCl₃, 300 MHz) 1.44 (s. 9 H). 1.58 (d, 6 H). 1.73-1.96 (m, 1 H). 2.17-2.31 (m, 1H). 2.38 (s. 1H). 2.64-2.93 (m. 5 H). 2.94-3.07 (m, 2 H). 3.45 (t, 3 H). 3.58-3.67 (m. I H), 7.02 (d, 2 H), 7.15-7.36 (m. 4 H). 7.47 (t. 1 H). 8.01 (d. 1H).

### 3d. 2-{[β-(4-(3-Mercapto-3-methyl-butyric acid)phenyl) ethyl]aminomethyl}-1-tetralone ester

The product of Example 3c (0.149 g, 0.29 mmol) was dissolved in dichloromethane (3 mL) and trifluoroacetic acid (3 mL) was added. The resulting solution was stirred for 15 minutes at room temperature. The solvent was evaporated, and the residue was dissolved in dichloromethane (10 mL). Water (5 mL) was added and pH was made basic with saturated sodium bicarbonate solution. Organic layer was separated and aqueous fraction was extracted with dichloromethane. The combined organic phase was washed with brine, dried over anhydrous sodium sulfate and concentrated in vacuo to give 0.098 g (82 %) of the title compound. ¹H NMR (CDCl₃, 300 MHz) 159 (s, 6 H), 1.84-2.03 (m, 1 H), 2.15-2.26 (m. 1H). 2.39 (s, 1 H), 2.82- 3.16 (m, 11 H), 7.06 (d, 2 H). 7.18-7.35 (m. 4 H), 7.49 (t. I H). 8.00 (1 H).

### 3e. 2-{[(β-(4-(3-S-Nitroso-3-methyl-butyric acid)phenyl) ethyl]aminomethyl}-1-tetralone ester hydrochloride

The product of Example 3d (0.081 g, 0.20 mmol) was dissolved in methanol (4 mL) and 1N HCl was added. A solution of sodium nitrite (0.045 g, 0.65 mmol) in water (0.25 mL) was added. After stirring for 15 minutes at room temperature an additional sodium nitrite (0.045 g. 0.65 mmol) in water (0.25 mL) was added. The reaction mixture was stirred for 15 more minutes, and was then extracted with dichloromethane. The organic layer was dried over anhydrous sodium sulfate and the solvent was evaporated to give 0.072 g (81 %) of the title compound as a green solid. ¹H NMR (CDCl₃, 300 MHz) δ:1.72-1.93 (m. 1 H), 2.09 (s, 6H). 2.18-2.30 (m. 1H). 2.84-3.11 (m, 1 H), 3.14-3.33 (m, 6 H). 3.36-3.57 (m. 4 H). 7.03 (d. 2 H), 7.18-7.42 (m. 4 H). 5.53 (t. I H). 7.94 (d. I H).

### Example 4

### 4-(2-methoxyphenyl)-α-[(1-naphthalenyloxy)methyl]-1-piperazineethyoxy-(3-S-nitroso-3-methyl-butyric acid] ester

### 4a. 3-Methyl-3-[2.4.6-trimethoxyphenyl methylthio)butyric acid

To a solution of 3-mercapto-3-methylbutyric acid (B.J. Sweetman et al. J. Med Chem., 14, 868 (1971)) (4.6 g. 34 mmol) in methylene chloride (250 mL) under nitrogen and cooled over ice/salt to 5°C (internal temperature) was added trifluoroacetic acid (82 g, 0.72 mol). No significant temperature rise was noted during the addition. To this was then added dropwise a solution of 2.4,6-trimethoxybenzyl alcohol (M.C. Munson et al., J Org. Chem., 57, 3013 (1992)) (6.45 g, 32 mmol) in methylene chloride (150 mL) such that the reaction temperature does not rise above 5°C. After the addition was complete, the mixture was stirred for an additional 5 minutes at 5°C and the volatiles were evaporated *in vacuo* (toluene or ethyl acetate can be used to assist in the removal of volatile material). The residue was partitioned between diethyl ether and water and the organic phase dried over anhydrous sodium sulfate, filtered and the volatile material evaporated *in vacuo.* The residue was treated with activated charcoal and recrystalised from diethyl ether/hexane. The product was isolated as an white solid in 70% yield (7 g) mp 103-105 °C. ¹H NMR (CDCl₃) δ: 6.12 (s. 2H), 3.80-3.85 (m, 11H), 2.74 (s, 2H). 1.47 (s. 6H). ¹³C NMR (CDCl₃) 173.9, 160.6, 158.6, 105.6, 90.5, 55.7, 55.3, 45.9, 43.6, 28.4, 21.0.

### 4b. 4-(2-methoxyphenyl)-α-[(1-naphthalenyloxy)methyl]1-Diperazineethyoxy-[3-(2,4,6-trimethyoxybenzylthio)-3-methyl-butyric acid] ester

Under a nitrogen atmosphere, 4-(2-methoxyphenyl)-α-[(1-naphthalenyloxy)methyl]-1-piperazineethanol (0.130 g, 0.35 mmol) was dissolved in anhydrous dimethylformamide (2 mL) and 4-dimethylaminopyridine (0.017 g. 0.14 mmol) was added, followed by the product of Example 4a (0.211 g. 0.69 mmol) and 1-ethyl-3-(3-dimethylaminopropyl)carbodiimide (0.132 g, 0.69 mmol). The resulting mixture was stirred 2 hours at room temperature and then 24 hours at 50°C. The solvent was evaporated *in vacuo* and the residue was purified by flash chromatography on silica gel eluting with hexane/ ethyl acetate (3:1) to (2:1) to give the title compound (0.133 g, 56 % yield). ¹HNMR (CDCl₃, 300 MHz) δ: 1.49-1.53 (d, 6 H, J = 2.42 Hz). 2.70- 2.84 - (m. 8H). 2.98-3.09 (m. 4 H), 3.75-3.85 (m. 11 H). 3.86 (s. 3 H). 4.31-4.36 (m. 2 H). 5.43-5.52 (m. 1 H), 6.08 (s. 2 H). 6.81-6 86 (m. 2 H), 6.90-6.93 (m. 2 H). 6.97- 7.01 (m. 1H). 7.33-7.7- (m, 4 H). 7.77-7.82 (m, 1 H), 8.23-8.27 (m. 1 H).

### 4c. 4-(2-methoxyphenyl)-α-[(1-naphthalenyloxy)methyl]-1-piperazineethyoxy-[3-mercapto-3-methyl-butyric acid] ester

The product of Example 4b (0.128 g, 0.186 mmol) was dissolved in methylene chloride (0.50mL), and then anisole (0.13 mL, 1.20 mmol), phenol (0.013 g.0.14 mmol), water (0.13 mL), and trifluoroacetic acid (0.80 mL. 10.4 mmol) were added. After 1 hour of stirring at room temperature, toluene (2 mL) was added and volatiles were evaporated. The residue was purified by flash chromatography on silica gel eluting with hexane/ ethyl acetate (2:1) to give the title compound (0.055 g, 60 % yield) as a solid. ¹H NMR (CDCl₃, 300 MHz) 1.49-1.53 (d. 6 H, J =2.42 Hz). 2.59 (s, 1 H). 2.69-2.86 (m. 8 H). 3.01-3.09 (m. 4 H). 3.86 (s. 3 H), 4.26-4.39 (m. 2 H). 5.53-5.63 (m. 1 H). 6.81-6.88 (d, 2 H. J =7.5 Hz). 6.90-6 95 (m, 2 H), 6.98-7.04 (m, 1 H), 7.34-7.40 (t. 1 H. J=7.5Hz). 7.43-7.78 (m, 3 H), 7.79-7.82 (m, 1 H), 8.23-8.26 (m, 1 H).

### 4d. 4-(2-methoxyphenyl)-α-[(1-naphthalenyloxy)methyl]-1-piperazineethyoxy-[3-S-nitroso-3-methyl-butyric acid] ester

The product of Example 4c (0.048 g, 0.097 mmol) was dissolved in methanol (5mL) and 1N solution of hydrochloric acid (1.5 mL) was added. The resulting mixture was cooled to 0°C and a solution of sodium nitrite 0.040 g, 0.058 mmol) in water (0.5mL) was added. After 1 hour stirring at 0°C the reaction mixture was extracted with methylene chloride, washed with brine and dried over anhydrous sodium sulfate. The solvent was evaporated *in vacuo* to give the title compound (0.045 g. 82 % yield) as a green solid. ¹HNMR(CDCl₃. 300 MHz) δ: 2.00 (s, 6 H), 3.38-3.50 (m. 13 H), 3.88 (s, 3 H). 4.31-4.40 (m, 2 H). 5.91 (s. 1 H). 6.79-6.95 (m, 5 H), 7.33-7.70 (m, 4 H), 7.79- 7.82 (m, 1 H). 8.09-8.12 (m. 1 H).

### Example 5

### 2-[4-(2-furoyl)piperazin-1-y1]-[4-(3-S-nitroso-3-methyl-butyric acid)]-6, 7-dimethyoxyquinoline amide

### 5a. 2-[4-(2-Furoyl)piperazin-1-yl]-[4-(3-(2,4,6-trimethyoxybenzylthio)-3-methylbutyric acid)]-6, 7-dimethyoxyquinazoline amide

Under a nitrogen atmosphere 2-[4-(2-furoyl)piperazin-1-yl]-amino-6. 7-dimethyoxyquinazoline (0.200 g, 0.52 mmol) was dissolved in anhydrous dimethylformamide (5 mL) and 4-dimethylaminopyridine (0.025 g. 0.21 mmol) was added, followed by the product of Example 4a (0.319 g. 1.04 mmol) and 1-ethyl-3-(3-dimethylaminopropyl)carbodiimide (0.199 g, 1.04 mmol). The resulting mixture was stirred at 50°C for 48 hours. The solvent was evaporated *in vacuo* and the residue was purified by flash chromatography on silica gel eluting with hexane/ethyl acetate (3:1) to (1:5) to give 0.072 g (20 % yield) of the title compound as a white solid. ¹H NMR (CDCl₃, 300 MHz) δ: 1.52 (s, 6 H). 2.88 (s, 1 H), 2.90 (s. 2 H), 2.96 (s, 1 H). 3.56 (s.6H), 3.72 (s. 3 H), 3.90-4.01 (m, 16 H), 6.48-6.52 (dd, 1 H, J = 1.69 and 3.32 Hz), 6.94 (s. 1 H), 7.01-7.05 (d. 1 H, J = 3.45 Hz), 7 19 (s, 1 H), 7.50-7.53 (m, 1 H).

### 5b. 2-[4-(2-Furoyl)piperazin-1-yl]:[4-(3-mecapto-3-methyl-butyric acid)]-6,7-dimethyoxyquinazoline amide

The product of Example 5a (0.160 g, 0.24 mmol) was dissolved in methylene chloride (0.67 mL), and then anisole (0.16 mL. 1.47 mmol), phenol (0.007 g. 0.047 mmol), water (0.16 mL), and trifluoroacetic acid (0.67 mL, 8.63 mmol) were added. After 45 minutes of stirring at room temperature toluene (5 mL) was added and volatiles were evaporated. The residue was purified by flash chromatography on silica gel eluting with methylene chloride/ methanol (30:1) to (15:1) to give the title compound (0.043 g, 36 % yield) as a solid. ¹H NMR (CDCl₃, 300 MHz) δ: 1..58 (s. 6 H), 2.45 (s, 1 H). 3.00 (s.2H), 3.87-3.94 (d. 6 H. J = 6.28 Hz). 3.92-4.06 (m. 8 H). 6.53-6.57 (dd. 1 H. J=1.68 and 3.41 Hz), 6.98 (s. 1 H), 7.15-7.18 (d. 1 H. J = 3.48 Hz), 7.49 (s. 1 H), 7.54-7.59 (m. 1 H).

### 5c. 2-[4-(2-Furoyl)piperazin-1-yl]-[4-(3-S-nitroso-3-methyl-butyric acid)]-6,7-dimethyoxyquinazoline amide

The product of Example 5b (0.036 g, 0.080 mmol) was dissolved in methanol and 1N solution of hydrochloric acid (1 mL) was added. The resulting mixture was cooled to 0°C and a solution of sodium nitrite (0.067 g, 0.97 mmol) in water (0.5 mL) was added. After 40 min. stirring at 0°C the reaction mixture was extracted with methylene chloride, washed with brine and dried over anhydrous sodium sulfate. The solvent was evaporated *in vacuo* to give the title compound (0.023 g. 55 % yield) as a green solid. ¹HNMR(CDCl₃. 300 MHz) δ: 2.12 (s. 6 H), 3.49 (s. 2 H). 3.85-3.99 (m. 14 H). 6.51-6.55 (dd. 1H, J = 1.74 and 3.45 Hz). 6.79-6.98 (m, 2 H). 7.06-7.09 (d, 1 H. J = 3.23 Hz). 7.54-7.58 (m. 1 H).

### Example 6

### 4-[2-(Dimethylamino)ethoxy]-2-methyl-5-(1-methylethyl)phenol-(3-S-nitroso-3-methyl-butyric acid) ester

### 6a. 4-[2-(Dimethylamino)ethoxyl-2-methyl-5-(1-methylethyl)phenol

4-[2-(Dimethylamino)ethoxy]-2-methyl-S-(1-methylethyl)phenol acetate ester (1.00 g. 3.20 mmol) was dissolved in methanol (10 mL) and sodium hydroxide (0.317 g. 7.92 mmol) was added. The reaction mixture was stirred at room temperature for 10 minutes, diluted with ethyl ether (10 mL) and washed with sodium bicarbonate solution. The organic layer was dried over anhydrous sodium sulfate, filtered and concentrated *in vacuo* to give the title compound (0.71 g. 93 % yield) as a white solid. ¹H NMR (CDCl₃. 300 MHz) δ: 1.10-1.13 (d. 6 H. J = 6.9 Hz), 2.19 (s, 3 H), 2.41 (s. 6 H). 2.80-2.85 (t. 2 H. J = 5.9 Hz). 3.19-3.26 (m. 1 H). 4.02-4.07 (t. 2 H, J=5.9Hz). 6.57- 6.59 (d. 2 H. J = 3.72 Hz).

### 6b. 4-[2-(Dimethylamino)ethoxy]-2-methyl-5-(1-methylethyl)phenol-[3-(2.4,6-trimethyoxybenzylthiol]-3-methyl-butyric acid) ester

Under a nitrogen atmosphere, the product of Example 6a (0.270 g. 1.14 mmol) was dissolved in anhydrous dimethylformamide (2 mL) and 4-dimethylaminopyridine (0.028g. 0.23 mmol) was added, followed by the product of Example 4a (0.418 g, 1.36 mmol) and 1-ethyl-3-(3-dimethylaminopropyl)carbodiimide (0.260 g, 1.36 mmol). The resulting mixture was stirred at 55°C for 24 hours. The solvent was evaporated *in vacuo* and the residue was purified by flash chromatography on silica gel, eluting with methylene chloride/ methanol (20:1) to give 0.232 g (39 % yield) of the title compound. ¹H NMR (CDCl₃, 300 MHz) δ: 1.14-1.18 (d. 6 H, J = 6.9 Hz), 1.59 (s, 6 H), 2.15 (s, 3 H), 2.35 (s. 6 H). 2.72-2.77 (t. 2 H. J = 5.9 Hz), 2.93-2.96 (m, 2 H), 3.23-3.28 (m, 1 H), 3.74-4.02 (m. 11 H), 4.03-4.07 (t. 2 H. J = 5.9 Hz). 6.11 (s. 2H). 6.67 (s, 1H). 6.81 (s, 1 H).

### 6c. 4-{2-(Dimethylamino)ethoxyl-2-methyl-5-(1-methylethyl)phenol-(3-mercapto-3-methyl-butyric acid) ester

The product of Example 6b (0.220 g. 0.42 mmol) was dissolved in methylene chloride (0.30mL) and anisole (0.22 mL. 2.02 mmol), phenol (0.022 g, 0.23 mmol), water (0.22 mL) and trifluoroacetic acid (1.0 mL. 13.0 mmol) were added. After 1 hour of stirring at room temperature, toluene (5 mL) was added and volatiles were evaporated. The residue was purified by flash chromatography on silica gel, eluting with methylene chloride/ methanol (20:1) to give the title compound (0.095 g. 64 % yield). ¹H NMR (CDCl₃, 300 MHz) δ: 1.14-1.16 (d. 6 H, J = 6.9 Hz). 1.58 (s, 6 H). 2.14 (s. 3 H). 2.40 (s. 1 H), 2.87-2.94 (m. 8 H), 3.14-3.20 (m, 1 H), 3.50-3.53 (m. 2 H). 4.31-4.34 (m. 2H). 6.67 (s, 1 H), 6.84 (s, 1 H).

### 6d. 4-[2-(Dimethylamino)ethoxyl-2-methyl-5-(1-methylethvl)phenol-(3-S-nitroso-3-methyl-butyric acid) ester

The product of Example 6c (0.035 g, 0.10 mmol) was dissolved in methanol (5mL) and 1N solution of hydrochloric acid (1 mL) was added. The resulting mixture was cooled to 0°C and a solution of sodium nitrite (0.014 g, 0.20 mmol) in water (0.7 mL) was added. After 20 minutes stirring at 0°C. an additional sodium nitrite (0.032 g. 0.46mmol) in water (0.7 mL) was added and the resulting mixture was stirred for 30 minutes. The reaction mixture was extracted with methylene chloride, washed with brine and dried over anhydrous sodium sulfate. The solvent was evaporated *in vacuo* to afford the product (0.028 g, 67% yield) as a green solid. ¹HNMR (CDCl₃, 300MHz) δ: 1.13-1.17 (d. 6 H, J = 6.9 Hz). 2.08-2.11 (m. 9 H). 2.95 (s, 6 H), 3.13-3.20 (m, 1 H). 3.45-3.51 (m. 4 H). 4.43-4.46 (m. 2 H). 6.23 (s. 1 H). 6.70 (s. 1 H), 6.76 (s, 1 H).

### Example 7

### 3-[[4,5-Dihydro-1-(3-S-nitroso-3-methyl butyloxy)-imidazol-2-yl)methyl](4-methylphenyl)aminolphenol-(3-S-nitroso-3-methyl-butyric acid) ester

### 7a.3-Mercapto-3-methyl butyl acetate

3-Mercapto-3-methyl butanol (Sweetman et al. J. Med. Chem. 14, 868 (1971) (5 g, 42 mmol) and pyridine (3.6 mL. 43 mmol) were dissolved in methylene chloride (50 mL) and cooled to -78°C. Acetyl chloride (3.1 mL. 43 mmol) was added dropwise. The solution was kept cold for 30 min then allowed to warm to room temperature. Stirring was continued for 1.5 hr. The reaction mixture was diluted with methylene chloride, washed with 1N HCl and brine, and dried over sodium sulfate. Evaporation of the solvent gave 6.6 g of the title compound which was used without further purification. ¹H-NMR (CDCl₃) δ: 4.25 (t, J = 7.1 Hz. 2H). 2.21 (s, 1H), 2.03 (s. 3H). 1.92 (t. J = 7.2 Hz, 2H). 1.41 (s, 3H).

### 7b. 3-Tetrahydropyranylthio-3-methyl butyl acetate

The product of Example 7a (6.6 g, 41 mmol), dihydropyran (4 mL. 44 mmol), and 4 N HCl/Et₂O (1 mL) were allowed to stand at room temperature for 24 hours. The volatiles were evaporated *in vacuo* to leave the title compound as a viscous oil which was used without further purification. ¹H-NMR (CDCl₃) δ: 4.97 (dd. J = 3.4 and 6.6 Hz. 1H). 4.24 (t. J = 7.1 Hz, 2H). 4.04-4.09 (mult, 1H), 3.46-3.52 (mult, 1H). 2.03 (s. 3H). 1.93 (t, J = 7.5 Hz. 2H). 1.42-1.88 (mult. 6H), 1.37 (s. 3H), 1.36 (s, 3H).

### 7c. 3-Tetrahydropyranylthio-6-methyl butanol

The product of Example 7b (800 mg. 3.3 mmol) and sodium bicarbonate (1.4 g. 16 mmol) were dissolved in methanol (10 mL) and stirred at room temperature for 18 hours. The reaction mixture was diluted with ether (30 mL) to precipitate the salts and filtered through Celite. Evaportation of the solvent and chromatography on silica gel eluting with 3:1 hexane/ethyl acetate gave 340 mg (51 %) of the title compound. ¹H-NMR (CDCl₃) δ: 4.92 (dd, J = 3.1 and 7.6 Hz, 1H), 4.05 (ddd; J = 4.0, 4.0. and 11.6 Hz, 1H), 3.81 (ddd; J = 6.3, 6.3. and 12.6 Hz. 1H). 3.78 (ddd; J = 6.3. 6.3, and 12.6 Hz, 1H), 3.49 (ddd; J = 3.8. 7.7. and 11.8 Hz, 1H). 1.79-1.89 (mult, 4H), 1.60-1.67 (mult, 4H), 1.56 (s. 3H). 1.55 (s. 3H). Anal calcd for C₁₀H₂₀O₂S: C; 58.78, H; 9.87. S; 15.69. Found C; 58.42, H; 9.73, S; 15.58.

### 7d. 3-[[4,5-Dihydro-1-(3-tetrahydropyranylthio-3-methyl butyloxy)-imidazol-2-yl)methyl](4-methylphenyl)amino]phenol-(3-tetrahydropyranylthio-3-methylbutyric acid) ester

The product of Example 7c (700 mg. 3.5 mmol) was dissolved in tetrahydrofuran (5 mL) and cooled to -78°C. To this solution was added 2.5 M BuLi (1.38 mL. 3.5 mmol), and the reaction mixture was stirred at -78°C for 20 minutes. A solution of 1.93 M phosgene in toluene (3.6 mL. 7.0 mmol) was cooled to -78°C and the cold solution of lithium alkokide was rapidly cannulated into the phosgene solution. The reaction mixture was stirred at -78°C for 30 minutes and then warmed to room temperature and stirred for 2 hours. The solution was filtered through a cotton plug and concentrated to give the chloroformate as a syrup. A slurry of 3-[[4,5-dihydro-1H-imidazol-2-yl)methyl](4-methylphenyl)amino]phenol hydrochloride (500 mg, 1.6 mmol) and triethylamine (650 µL. 4.7 mmol) in methylene chloride (10 mL) was cooled to to -78°C. The chloroformate was dissolved in methylene chloride (4 mL) and this solution was added to the slurry. The resulting reaction mixture was stirred at -78°C for 30 minutes and was then warmed to room temperature and stirred for 20 hours. The reaction mixture was diluted with methylene chloride and then washed successively with 0.1 N HCl. saturated aqueous sodium bicarbonate, and brine; followed by drying over sodium sulfate. Evaporation of the solvent and chromatography on silica gel eluting with 2:1 hexane/ethyl acetate gave 540 mg (46 %) of the title compound. ¹H-NMR (CDCl₃) δ: 7.18 (d, J = 8.6 Hz. 2H). 7.13 (d, J = 8.0 Hz, 2H), 7.12 (t, J = 8.2 Hz, 1H), 6.57-6.62 (mult, 2H). 6.51 (t. J = 2.2 Hz, 1H), 4.94-4.99 (mult, 2H), 4.89 (s, 2H), 4.38 (t, J = 7.3 Hz, 2H), 4.32 (t. J = 7.1 Hz. 2H), 4.03-4.08 (mult, 2H), 3.79 (s, 4H). 3.46-3.52 (mult, 2H), 2.32 (s, 3H). 1.51-2.05 (mult. 16H).

### 7e. 3-[[4.5-Dihydro-1-(3-thiol-3-methyl butyloxy)-imidazol-2-yl)methyl](4-methylphenyl)amino]phenol-(3-thiol-3-methyl-butyric acid) ester

The product of Example 7d (400 mg. 0.54 mmol), mercaptoethanol (760 µL. 10 mmol), and 4 N HCl in ether (250 µL, 1 mmol) were kept at room temperature for 24 hours. The reaction mixture was diluted with ethyl acetate and then washed with saturated aqueous sodium bicarbonate, water, and brine, and then dried over sodium sulfate. Hydrochloric acid was added and the solvent was evaporated to leave a syrup. The syrup was triturated with ethanol and ether. Decantation of the solvents and subjecting the residue to high vacuum overnight afforded 130 mg of solid. The solid was chromatographed on silica gel eluting with 3:1 hexane/ethyl acetate to give 30 mg (10 %) of the title compound. ¹H-NMR (CDCl₃) δ: 7.18 (d. J = 8.6 Hz 2H). 7.14 (d. J = 7.9 Hz, 2H). 7.13 (t. J = 8.2 Hz. 1H). 6.61 (dd, J = 2.4 and 8.3 Hz. 1H). 6.59 (dd. J = 2.1 and 7.9 Hz. 1H). 6.52 (t. J = 2.2 Hz. 1H). 4.90 (s. 2H), 4.41 (t. J = 7.3 Hz, 2H). 4.35 (t. J = 7.0 Hz. 2H). 3.80 (s. 4H), 2.33 (s. 3H). 2.02 (t, J = 7.1 Hz, 2H). 1.97 (t, J = 7.1 Hz. 2H). 1.76 (s, 1H), 1.75 (s. 1H). 1.43 (s. 12H).

### 7f. 3-[[4.5-Dihydro-1-(3-S-nitroso-3-methyl butyloxy)-imidazol-2-yl)methyl](4-methylphenyl)amino]phenol-(3-S-nitroso-3-methyl-butyric acid) ester

The product of Example 7e (18 mg. 0.033 mmol) was dissolved in dimethylforamide (200 µL) and 4 N HCl in ether (25 µL, 0.1 mmol) was added. The reaction mixture was cooled to 0°C and tert-butyl nitrite (12 µL, 0.12 mmol) was added and then the reaction mixture was stirred at for 0°C for 20 minutes. The solvent was - evaporated *in vacuo* and the solid residue obtained was azetroped with chloroform to afford the title compound as a foam. ¹H-NMR (DMSO-d₆) δ: 7.09 (d. J = 8.0 Hz. 1H). 6.89 (d. J = 8.2 Hz, 1H), 6.61-6.72 (mult, 6H). 5.10 (br s, 2H). 4.44 (t. J = 6.7 Hz. 2H). 4.38 (t. J = 6.7 Hz, 2H), 4.00-4.10 (mult, 2H), 3.89-4.00 (mult. 2H). 2.65 (t. J = 6.5 Hz. 2H). 2.62 (t, J = 6.6 Hz, 2H), 2.30 (s, 3H), 1.92 (s, 6H), 1.89 (s, 6H).

### Example 8

### 4-[2-(Dimethylamino)ethoxy]-2-methyl-5-(1-methylethyl)phenol-(4-O-nitro-3-butyric acid) ester

### 8a. 4-[2-(Dimethylamino)ethoxy]-2-methyl-5-(1-methylethyl)phenol-(4-bromobutyric acid) ester

4-Bromobutyric acid (1 g. 6 mmol) and triethylamine (836ml- 6 mmol) was dissolved in ethyl acetate (40 ml) and cooled to 0 °C. Triphosgene (309 mg. 1.04 mmol) was added all in one portion and the reaction was stirred at 0 °C for 15 minutes then warmed to room temperature with continued stirring for 1 hour. The precipitate which formed was removed by filtration and the filtrate was concentrated by rotary evaporation to afford a light beige oil which was used without further purification. The product of Example 6a (395 mg. 1.78 mmol) was dissolved in dry acetonitrile (10 ml) and added to the preformed symmetrical anhydride. Scandium triflate (20 mg. 0.04 mmol) was added and the reaction mixture was stirred at room temperature for 2 hours after which time additional triethylamine (203 mg. 2 mmol) was added and stirring was continued for an additional 4 hours. The reaction was concentrated in vacuo and the residue partioned between ethyl acetate and phosphate buffer (pH 7.1). The organic phase was dried over sodium sulfate and the solvent evaportated in vacuo to afford a brown oil. The oil was chromatographed on silica gel eluting with 9:1 methylene chloride/methanol to give 152 mg (22 %) of the title compound as a light beige oil. ¹H-NMR (CDCl₃) δ: 6.84 (s. 1H), 6.69 (s. 1H), 4.35 (dd. J = 4.7 Hz and 4.7 Hz. 2H). 3.55 (t, J = 6.4 Hz. 2H), 3.41 (dd. J = 4.7 Hz and 4.7 Hz, 2H). 3.18 (m. 1H), 2.90 (s. 6H). 2.79 (t. J = 7.2 Hz, 2H). 2.30 (m. 2H), 2.13 (s, 3H). 1.18 (d. J = 6.9 Hz. 6H).

### 8b. 4-[2-(Dimethylamino)ethoxy]-2-methyl-5-(1-methylethyl)phenol-(4-iodo-butyric acid) ester

The product of Example 8a (150 mg. 0.39 mmol). sodium iodide (116 mg. 0.77 mmol) and acetone (15 ml) were stirred at 50 °C for 3 hours. The solvent was evaporated in vacuo and the residue partioned between methylene chloride and and phosphate buffer (pH 7.1). The organic phase was dried over sodium sulfate and the solvent evaporated in vacuo to afford 131 mg of the crude title compound which was imidiately carried on to the next step. ¹H-NMR (CDCl₃) δ: 6.82 (s, 1H), 6.69 (s, 1H), 4.23 (dd. J = 5.4 Hz and 5.4 Hz. 2H), 3.32 (t, J = 6.6 Hz. 2H), 3.23 (m, 1H), 3.04 (dd, J = 5.4 Hz and 5.4 Hz. 2H). 2.72 (t. J = 7.2 Hz, 2H), 2.56 (s, 6H), 2.28 (m, 2H), 2.12 (s, 3H). 1.18 (d, J = 6.9 Hz. 6H).

### 8b. 4-[2-(Dimethylamino)ethoxyl]-2-methyl-5-(1-methylethyl)phenol-(O-nitrobutyric acid) ester

The product of Example 8b (130 mg, 0.30 mmol) was dissolved in acetonitrile (5 ml) and silver nitrate (102 mg, 0.60 mmol) was added and the reaction mixture was left over night at tomm temperature. The reaction mixture was filtered through a PTFE membrane and the solvent evaporated in vacuo. The residue was chromatographed on silica gel eluting with 9:1 methylene chloride/methanol to give 19 mg (17 %) of the title compound as an oil which was solubilized in a small volume of ethyl acetate and precipitated by the addition of ether. mp: 105-107 °C MS: (DCI/NH₃): m/z = 369 (M+H)⁻¹H-NMR (CDCl₃) δ: 6.84 (s. 1H), 6.71 (s. 1H), 4.49 (t, J = 6.2 Hz. 2H), 4.36 (dd. J = 4.5 Hz and 4.5 Hz, 2H). 3.56 (dd, J = 4.5 Hz and 4.5 Hz, 2H). 3.19 (m, I H). 2.99 (s. 6H). 2.72 (t. J = 7.2 Hz. 2H). 2.19 (m, 1 H), 2.11 (s, 3H). 1.17 (d. J = 6.6 Hz, 6H).

### Example 9

### In Vivo Comparative Erectile Responses

Male New Zealand white rabbits weighing 2.5 kg were used as the animal model. Animals were first relaxed with an i.m. injection of 25 mg/kg ketamine prior to anesthesia with a bolus i.v. injection of 10 mg/kg Profol and maintained with i.v. infusion at 0.5 mg/kg/min. Ventilation of the animals was performed with 1% halothane plus 0.8 L/min O₂ and 1 L/min N,O. A 22 gauge angiocatheter was placed in the femoral artery for measurement of systemic blood pressure. A dorsal incision was made in the penis and the corpora cavemosa exposed and cannulated with a 21 gauge butterfly needle to measure intracavernosal pressure.

Drugs at various concentrations were delivered intracavernosally at a volume of 150 µl through a 25 gauge needle. A 150 µl solution of a mixture of papaverine (30 mg/kg), phentolamine (1 mg/kg) and prostaglandin El (10 µg/ml) (pap/phent/PGE1) was injected in the corpora as a standard solution for comparison with the response of yohimbine, the compound of Example 1, the compound of Example 2. and the combination of yohimbine and the compound of Example 1. This pap/phent/PGEI mixture is considered to cause a maximal erection-inducing effect.

As shown in Figure 1, yohimbine dose dependently induced erectile response in the anesthetized rabbit. A 500 µg dose of the compound of Example 1 was able to induce near maximal response relative to the standard dose of pap/phent/PGE1. A combination of the submaximal dose of yohimbine (150 µg) and the compound of Example 1 (500 µg) also induced maximum erectile response. Yohimbine at both the submaximal and maximal efficacy doses produced very short duration of action (Figure 2). The compound of Example 1 produced a much longer duration of action. The duration of action is potentiated bv a combination of the compound of Example I and yohimbine which is longer than the sum of the duration of each of these compounds alone (Figure 2).

Figure 3 shows that the compound of Example 2 at the 500 µg dose is equipotent to the standard dose of pap/phent/PGE 1. A higher dose of the compound of Example 2 (1 mg) is at least equal to or more efficacious that the standard dose of the pap/phent/PGEI mixture. Figure 4 shows that the compound of Example 2 has the advantage of producing longer duration of action compared to yohimbine. Figure 5 demonstrates that a dose (500 µg) of the compound of Example 2 effective in the erectile response did not produce any effect on systemic blood pressure upon intracavernosal injection. However, a standard dose of the mixture of pap/phent/PGEI produced a significant decrease in systemic blood pressure upon intracavernosal injection. suggesting that the compound of Example 2 lacks this side effect.

Figure I shows the percent peak erectile response *in vivo* compared to that produced by 150 µl of pap/phent/PGEI (30 mg/ml: 1 mg/ml: 10 µg/ml) in the anesthetized rabbit following the intracavernosal injection of 150 µl of yohimbine (150 µg. 500 µg), the compound of Example 1 (500 µg), and a combination of yohimbine (150 µg) and the compound of Example I (500 µg). The ordinate is the percent response of intracavernosal pressure relative to that produced by pap/phent/PGEI and the abscissa indicates the various drugs given.

Figure 2 shows the duration of the erectile response *in vivo* in the anesthetized rabbit upon intracavernosal administration of yohimbine (150 µg. 500 µg), the compound of Example 1 (500 µg), and a combination of yohimbine (150 µg) and the compound of Example 1 (500 µg). The ordinate indicates the various drugs given and the abscissa is the duration in minutes.

Figure 3 shows the percent peak erectile response *in vivo* compared to that produced by 150 µl of pap/phent/PGEI (30 mg/ml: 1 mg/ml: 10 µg/ml) in the anesthetized rabbit following the intracavemosal injection of 150 gl of yohimbine (150 µg, 500 µg and 1 mg) and the compound of Example 2 (500 µg. 1 mg). The ordinate is the percent response of intracavernosal pressure relative to that produced by pap/phent/PGEI and the abscissa indicates the various doses of yohimbine and Example 2 given.

Figure 4 shows the duration of the erectile response *in vivo* in the anesthetized rabbit upon intracavernosal administration of yohimbine (150 µg, 500 ug and 1 mg) and the compound of Example 2 (500 µg and 1 mg). The ordinate indicates the various doses of yohimbine and the compound of Example 2 given and the abscissa is the duration in minutes.

Figure 5 compares the effects of intracavernosal injections of the compound of Example 2 (500 µg) and the standard mixture of pap/phent/PGEI on systemic blood pressure in the anesthetized rabbit.

Figure 6 shows the percent peak erectile response *in vivo* compared to that produced by 150 µl of pap/phent/PGE 1 (30 mg/ml: 1 mg/ml: 10 µg/ml) in the anesthetized rabbit following the intracavernosal injection of moxisylyte (1 mg) and the compound of Example 6 (1 mg). The ordinate is the percent response of intracavernosal pressure relative to that produced by pap/phent/PGE1 and the abscissa indicates the dose of moxisylyte and the compound of Example 6 given.

Figure 7 shows the duration of the erectile response *in vivo* in the anesthetized rabbit upon intracavernosal administration of moxisylyte (1 mg) and the compound of Example 6 (1 mg). The ordinate indicates the dose of moxisylyte and the compound of Example 6 the abscissa is the duration in minutes.

## Claims

1. A nitrosated and/or nitrosylated α-adrenergic receptor antagonist wherein the α-adrenergic receptor antagonist is a compound that has been nitrosated and/or nitrosylated through an oxygen atom, a nitrogen atom or a sulfur atom which is a compound of formula III, or formula VIII:
wherein the compound of formula III is: 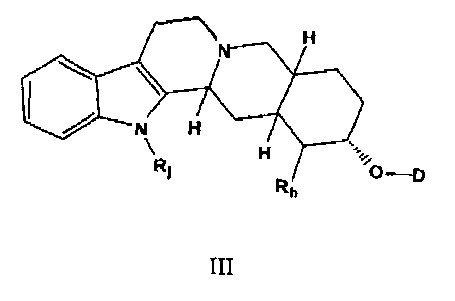 wherein Rₕ is hydrogen, -C(O)-O R_{d} or -C(O)-X;
X is (1)-Y-(C(R_{c})(R_{f}))ₚ-G-((C(R_{c})(R_{f}))ₚ-T-Q; or
(2) 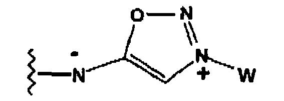 wherein Gᵢ is a covalent bond, -T-C-(O)-, -C(O)-T, -C(Y-C(O)-Rₘ)-; Rₘ is heteroaryl or heterocyclic ring; W is a heterocyclic ring or NR; R'ᵢ where in Rᵢ and R'ᵢ are each independently lower alkyl, aryl or alkenyl; and wherein Rⱼ is -D or -(O)C-R_{d};
wherein a is an integer of 2 or 3:
D is (i) -NO;
(ii) -NO₂;
(iii) -C(R_{d})-O-C(O)-Y-Z-(C(Rₑ)(R_{f}))ₚ-T-Q;
(iv) -C(O)-T¹-Z-(C(Rₑ)(R_{f})ₚ-T²-Q; or
(v) -C(O)-T(C(R_{y})(R_{z}))ₚ;
wherein R_{d} is hydrogen, lower alkyl, cycloalkyl, aryl, alkylaryl or heteroaryl; Y is oxygen, sulfur, or NRᵢ, Rᵢ is hydrogen or lower alkyl; R_{c} and R_{f} are each independently hydrogen, lower alkyl, cycloalkyl, aryl, heteroaryl, arylalkyl, amino, alkylamino, amido, alkylamido, diakylamino, or carboxy, or Rₑ and R_{f} taken together are carbonyl, cycloalkyl or bridged cycloalkyl; p is an integer from 1 to 6; T is oxygen, sulfur or nitrogen; Z is a covalent bond, alkyl, cycloalkyl, aryl, heteroaryl, arylalkyl or arylheterocyclic ring; Q is -NO or -NO₂; T¹ and T² are each independently T; R_{y} and R_{z} are each independently -T¹-(C(Rₑ)(R_{f}))ₚ-G-(C(Rₑ)(R_{f}))ₚ-T²-Q; G is a covalent bond, -T-C(O)-, -C(O)-T, or Y;
and wherein the compound of formula VIII is: 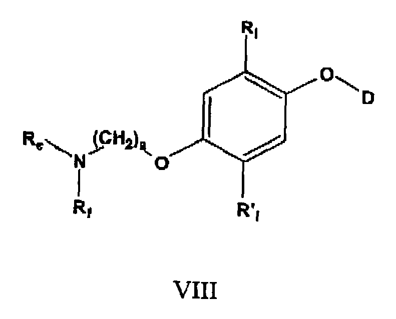 wherein a, Rᵢ, Rⱼ, Rₑ, R_{f} and D are defined as above.

2. The nitrosated and/or nitrosylated α-adrenergic receptor antagonist of claim 1,
wherein the nitrosated and/or nitrosylated α-adrenergic receptor antagonist is a nitrosated and/or nitrosylated moxisylyte.

3. The nitrosated and/or nitrosylated α-adrenergic receptor antagonist of claim 1,
wherein the nitrosated and/or nitrosylated α-adrenergic receptor antagonist is a nitrosated and/or nitrosylated yohimbine.

4. A composition comprising the nitrosated and/or nitrosylated α-adrenergic receptor antagonist of any of claims 1-3 and a pharmaceutically acceptable carrier.

5. A composition comprising the nitrosated and/or nitrosylated α-adrenergic receptor antagonist of any of claims 1-3, and at least one compound that donates, transfers or releases nitric oxide, elevates endogenous synthesis levels of nitric oxide or stimulates endogenous nitric oxide synthesis, which is L-asginine, an S-nitrosothiol or
(i) a compound comprising at least one ON-O-, ON-N- or ON-C- group;
(ii) a compound comprising at least one O₂N-O- O₂N-N- O₂N-S- or O₂N-C- group;
(iii) a N-oxo-N-nitrosoamine comprising an R₁R₂-N(O-M⁺)-NO group, wherein R₁ and R₂ are each independently a polypeptide, an amino acid, a sugar, a modified or unmodified oligonucleotide, a straight or branched, saturated or unsaturated, substituted or unsubstituted, aliphatic or aromatic hydrocarbon; or a heterocyclic compound; and M⁺ is a metal cation; or
(iv) a thionitrate having the structure R₁₀-S-NO₂ wherein R₁₀ is a polypeptide, an amino acid, a sugar, a modified or unmodified oligonucleotide, a straight or branched, saturated Or unsaturated, substituted or unsubstituted, aliphatic or aromatic hydrocarbon; or a heterocyclic compound*

6. The composition of claim 5, wherein the S-nitrosothiol is S-nitroso-N-acetylcysteine, S-nitroso-captopril, S-nitroso-homocysteine, S-nitroso-cysteine or S-nitroso-glutathione.

7. The composition of claim 5, wherein the S-nitrosothiol is
(i) CH₃(C(Rₑ)(R_{f}))ₓSNO;
(ii) HS(C(Rₑ)(R_{f}))ₓSNO;
(iii) ONS(C(Rₑ)(R_{f})ₓB; or
(iv) H₂N-CH(CO₂H)-(CH₂)ₓ-C(O)NH-CH(CH₂SNO)-C(O)NH-CH₂-CO₂H
wherein x equals 2 to 20; Rₑ and R_{f} are each independently selected from hydrogen, lower alkyl, cycloalkyl, aryl, heteroaryl, arylalkyl, alkylamino, or dialkylamino, or Rₑ and R_{f} taken together are carbonyl, cycloalkyl or bridged cycloalkyl; and B is flouro, alkoxy, cyano, carboxamido, cycloalkyl, arylalkoxy, alkylsulfinyl, arylthio, alkylamino, dialkylamino, hydroxy, carbamoyl, N-alkylcarbamoyl, N,N-dialkylcarbamoyl, amino, hydroxyl, carboxyl, hydrogen, nitro or aryl.

8. The compound of claim 5, wherein the compound comprising at least one ON-O-, ON-N- or ON-C- group is a ON-N- polypeptide, an ON-C- polypeptide, an ON-N-amino acid, an ON-C- amino acid, a ON-N- sugar, a ON-C- sugar, a ON-N- modified or unmodified oligonucleotide, a ON-C- modified or unmodified oligonucleotide, a straight or branched, saturated or unsaturated, aliphatic or aromatic ON-O- hydrocarbon; a straight or branched, saturated or unsaturated, substituted or unsubstituted, aliphatic or aromatic ON-N-hydrocarbon; a straight or branched, saturated or unsaturated, substituted or unsubstituted, aliphatic or aromatic ON-C- hydrocarbon; a ON-N- heterocyclic compound, or a ON-C-heterocyclic compound.

9. The compound of claim 5, wherein the compound comprising at least one O₂N-O-, O₂N-N-, O₂N-S- or O₂N-C- group is an O₂N-O- polypeptide, an O₂N-O- amino acid, an O₂N-O- sugar, an O₂N-O- modified or unmodified oligonucleotide, a straight or branched, saturated or unsaturated, substituted or unsubstituted, aliphatic or aromatic O₂N-O- hydrocarbon; an O₂N-O- heterocyclic compound; an O₂N-N- polypeptide, an O₂N-N- amino acid, an O₂N-N-sugar, an O₂N-N- modified or unmodified oligonucleotide, a straight or branched, saturated or unsaturated, substituted or unsubstituted, aliphatic or aromatic O₂N-N- hydrocarbon; an O₂N-N-heterocyclic compound; an O₂N-S- polypeptide, an O₂N-S- amino acid, an O₂N-S- sugar, an O₂N-S- modified or unmodified oligonucleotide, a straight or branched, saturated or unsaturated, substituted or unsubstituted, aliphatic or aromatic O₂N-S- hydrocarbon; an O₂N-S- heterocyclic compound; an O₂N-C polypeptide, an O₂N-C amino acid, an O₂N-C- sugar, an O₂N-C-modified or unmodified oligonucleotide, a straight or branched, saturated or unsaturated, substituted or unsubstituted, aliphatic or aromatic O₂N-C- hydrocarbon; or an O₂N-C-heterocyclic compound.

10. The composition of claim 5, wherein the compound that donates, transfers or releases nitric oxide is a nitrate having the structure R₁₀-S-NO₂ wherein R₁₀ is a polypeptide, an amino acid, a sugar, a modified or unmodified oligonucleotide, a straight or branched, saturated or unsaturated, substituted or unsubstituted, aliphatic or aromatic hydrocarbon; or a heterocyclic compound.

11. A compound according to claim 1 which is (9S, 8R)-5,8-bis(methoxycarbonyl)6,7,8,9,10,11α,6α,7α-decahydrobenzo(4,3-γ)indolo(2,3-β)quinolizin-9-yl-3-methyl-3-(nitrosothio)butanoate and acid addition salts thereof.

12. A compound according to claim 1 which is 4-(2-(dimethylamino)ethoxy)-2-methyl-5-(1-methylethyl)phenol-3-methyl-3-(nitrosothio)butanoate and acid addition salts thereof.

13. A composition according to claim 5 comprising an α-adrenergic receptor antagonist selected from yohimbine and moxisylyte and a compound that donates, transfers or releases nitric oxide or elevates endogenous synthesis levels of nitric oxide, is L-arginine, an S-nitrosothiol or
(i) a compound comprising at least one ON-O-, ON-N- or ON-C- group;
(ii) a compound comprising at least one O₂N-O-, O₂N-N-, O₂N-S- or O₂N-C- group;
(iii) a N-oxo-N-nitrosoamine comprising an R₁R₂-N(O-M⁺)-NO group, wherein R₁ and R₂ are each independently a polypeptide, an amino acid, a sugar, a modified or unmodified oligonucleotide, a straight or branched, saturated or unsaturated, substituted or unsubstituted, aliphatic or aromatic hydrocarbon; or a heterocyclic compound; and M⁺ is a metal cation; or
(iv) a thionitrate having the structure R₁₀-S-NO₂ wherein R₁₀ is a polypeptide, an amino acid, a sugar, a modified or unmodified oligonucleotide, a straight or branched, saturated or unsaturated, substituted or unsubstituted, aliphatic or aromatic hydrocarbon; or a heterocyclic compound.

14. The composition of claim 13, wherein the α-adrenergic receptor antagonist is moxisylyte.

15. The composition of claim 13, wherein the α-adrenergic receptor antagonist is yohimbine.

16. The composition of claim 13, wherein the S-nitrosothiol is S-nitroso-N-acetylcysteine, S-nitroso-captopril, S-nitroso-homocysteine, S-nitroso-cysteine or S-nitroso-glutathione.

17. The composition of claim 13, wherein the S-nitrosothiol is
(i) CH₃(C(Rₑ)(R_{f}))ₓSNO;
(ii) HS(C(Rₑ)(R_{f}))ₓSNO;
(iii) ONS(C(R_{c})(R_{f}))ₓB; or
(iv) H₂N-CH(CO₂H)-(CH₂)ₓ-C(O)NH-CH(CH₂SNO)-C(O)NH-CH₂-CO₂H
wherein x equals 2 to 20; Rₑ and R_{f} are each independently selected from hydrogen, lower alkyl, cycloalkyl, aryl, heteroaryl, arylalkyl, alkylamino, or dialkylamino, or Rₑ and R_{f} taken together are carbonyl, cycloalkyl or bridged cycloalkyl; and B is flouro, alkoxy, cyano, carboxamido, cycloalkyl, arylalkoxy, alkylsulfinyl, arylthio, alkylamino, dialkylamino, hydroxy, carbamoyl, N-alkylcarbamoyl, N,N-dialkylcarbamoyl, amino, hydroxyl, carboxyl, hydrogen, nitro or aryl.

18. The composition of claim 13, wherein the compound comprising at least one ON-O-, ON-N- or ON-C- group is a ON-N- polypeptide, an ON-C- polypeptide, an ON-N-amino acid, an ON-C- amino acid, a ON-N- sugar, a ON-C- sugar, a ON-N- modified or unmodified oligonucleotide, a ON-C- modified or unmodified oligonucleotide, a straight or branched, saturated or unsaturated, aliphatic or aromatic ON-O- hydrocarbon; a straight or branched, saturated or unsaturated, substituted or unsubstituted, aliphatic or aromatic ON-N-hydrocarbon; a straight or branched, saturated or unsaturated, substituted or unsubstituted, aliphatic or aromatic ON-C- hydrocarbon; a ON-N- heterocyclic compound, or a ON-C-heterocyclic compound.

19. The composition of claim 13, wherein the compound comprising at least one O₂N-O-, O₂N-N-, O₂N-S or O₂N-C- group is an O₂N-O- polypeptide, an O₂N-O- amino acid, an O₂N-O- sugar, an O₂N-O modified or unmodified oligonucleotide, a straight or branched, saturated or unsaturated, substituted or unsubstituted, aliphatic or aromatic O₂N-O- hydrocarbon; an O₂N-O- heterocyclic compound; an O₂N-N- polypeptide, an O₂N-N- amino acid, an O₂N-N-sugar, an O₂N-N- modified or unmodified oligonucleotide, a straight or branched, saturated or unsaturated, substituted or unsubstituted, aliphatic or aromatic O₂N-N- hydrocarbon; an O₂N-N-heterocyclic compound; an O₂N-S- polypeptide, an O₂N-S- amino acid, an O₂N-S- sugar, an O₂N-S- modified or unmodified oligonucleotide, a straight or branched, saturated or unsaturated, substituted or unsubstituted, aliphatic or aromatic O₂N-S- hydrocarbon; an O₂N-S- heterocyclic compound; an O₂N-C- polypeptide, an O₂N-C- amino acid, an O₂N-C- sugar, an O₂N-C-modified or unmodified oligonucleotide, a straight or branched, saturated or unsaturated, substituted or unsubstituted, aliphatic or aromatic O₂N-C- hydrocarbon; or an O₂N-C-heterocyclic compound.

20. The composition of any one of claim 13 to 15, wherein the compound that donates, transfers or releases nitric oxide or elevates endogenous synthesis levels of nitric oxide, is a nitrate having the structure R₁₀-S-NO₂ wherein R₁₀ is a polypeptide, an amino acid, a sugar, a modified or unmodified oligonucleotide, a straight or branched, saturated or unsaturated, substituted or unsubstituted, aliphatic or aromatic hydrocarbon; or a heterocyclic compound.

21. A composition comprising yohimbine and L-arginine.

22. The composition of any of claims 13 to 21, further comprising a pharmaceutically acceptable carrier.

23. The composition of claim 4 or claim 21, wherein the composition is in an orally administrable form.

24. The composition of claim 23, wherein the orally administrable form is a solid dosage form or a liquid dosage form.

25. The composition of claim 24, wherein the solid dosage form is a capsule, a tablet, a powder, a granule or a gel.

26. The composition of claim 24, wherein the liquid dosage form is an emulsion, a solution, a suspension, a syrup, or an elixir.

27. The composition of claim 4 or claim 22, wherein the composition is in a topically administrable form.

28. A kit comprising the composition of any one of claim 5 or claim 16 to 21.

29. A kit comprising yohimbine and L-arginine.

30. The kit of claim 29, wherein the yohimbine and L-arginine are separate components in the kit.

31. The use of a composition as claims in any one of claims 4 or 16 to 21 in the manufacture of a medicament for the treating human impotence in an individual in need thereof.

32. The use of the nitrosated and/or nitrosylated α-adrenergic receptor antagonist of claim 1 and a compound that donates, transfers or releases nitric oxide, elevates endogenous synthesis levels of nitric oxide or stimulates endogenous nitric oxide synthesis as defined in claim 5 in the manufacture of a medicament for the treating human impotence in an individual in need thereof.

33. The use as claimed in claim 31 or claim 32 wherein the human impotence is male impotence or female impotence.

34. The use of yohimbine and L-arginine in the manufacture of a medicament for the treating human impotence in an individual in need thereof.

35. The use of claim 34, wherein the medicament is for oral or topical administration.

## Patentansprüche

1. Nitrosierter und/oder nitrosylierter α-adrenerger Rezeptorantagonist, wobei der α-adrenerge Rezeptorantagonist eine Verbindung ist, die durch ein Sauerstoffatom, ein Stickstoffatom oder ein Schwefelatom nitrosiert und/oder nitrosyliert wurde, und eine Verbindung der Formel III oder der Formel VIII ist:
wobei die Verbindung der Formel III: 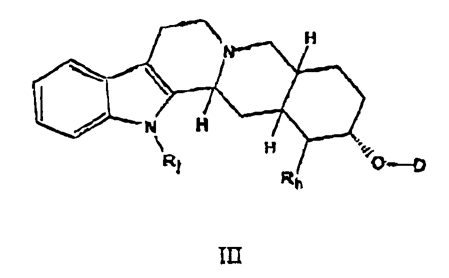
ist, worin Rₕ Wasserstoff, -C(O)O-R_{d} oder -C(O)-X ist;
X (1)-Y-(C(Rₑ)(R_{f}))ₚ-G-((C(Rₑ)(R_{f}))ₚ-T-Q oder
(2) 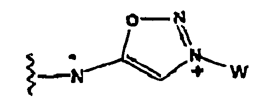 ist,
worin Gᵢ eine kovalente Bindung, -T-C-(O)-, -C(O)-T, -C(Y-C(O)-Rₘ)- ist; Rₘ Heteroaryl oder ein heterocyclischer Ring ist; W ein heterocyclischer Ring oder NRᵢR'ᵢ ist; worin Rᵢ und R'ᵢ jeweils unabhängig Niederalkyl, Aryl oder Alkenyl sind; und worin Rⱼ -D oder -(O)C-R_{d} ist;
worin a eine ganze Zahl von 2 oder 3 ist:
D (i) -NO;
(ii) -NO₂;
(iii) -C(R_{d})-O-C(O)-Y-Z-(C(Rₑ)(R_{f})ₚ-T-Q ;
(iv) -C(O)-T¹-Z-(C(Rₑ)(R_{f})ₚ-T²-Q oder
(v) -C(O)-T(C(R_{y})(R_{z}))ₚ ist;
worin R_{d} Wasserstoff, Niederalkyl, Cycloalkyl, Aryl, Alkylaryl oder Heteroaryl ist; Y Sauerstoff, Schwefel oder NRᵢ ist; Rᵢ Wasserstoff oder Niederalkyl ist; Rₑ und R_{f} jeweils unabhängig Wasserstoff, Niederalkyl, Cycloalkyl, Aryl, Heteroaryl, Arylalkyl, Amino, Alkylamino, Amido, Alkylamido, Dialkylamino oder Carboxy sind; oder Rₑ und R_{f} zusammen Carbonyl, Cycloalkyl oder verbrücktes Cycloalkyl sind; p eine ganze Zahl von 1 bis 6 ist; T Sauerstoff, Schwefel oder Stickstoff ist; Z eine kovalente Bindung, Alkyl, Cycloalkyl, Aryl, Heteroaryl, Arylalkyl oder ein arylheterocyclischer Ring ist; Q -NO oder -NO₂ ist; T' und T² jeweils unabhängig T sind; R_{y} und R_{z} jeweils unabhängig -T¹-(C(Rₑ)(R_{f}))ₚ-G-(C(Rₑ)(R_{f}))ₚ-T²-Q sind; G eine kovalente Bindung, -T-C(O)-, -C(O)-T oder Y ist;
und wobei die Verbindung der Formel VIII: 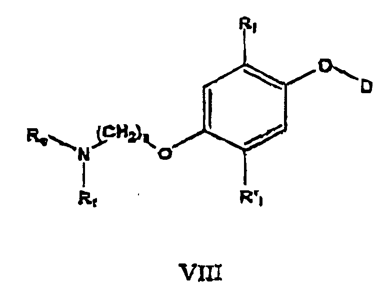 ist, worin a, Rᵢ, Rⱼ, Rₑ, R_{f} und D wie oben definiert sind.

2. Nitrosierter und/oder nitrosylierter α-adrenerger Rezeptorantagonist nach Anspruch 1, wobei der nitrosierte und/oder nitrosylierte α-adrenerge Rezeptorantagonist nitrosiertes und/oder nitrosyliertes Moxisylyt ist.

3. Nitrosierter und/oder nitrosylierter α-adrenerger Rezeptorantagonist nach Anspruch 1, wobei der nitrosierte und/oder nitrosylierte α-adrenerge Rezeptorantagonist nitrosiertes und/oder nitrosyliertes Yohimbin ist.

4. Zusammensetzung, umfassend den nitrosierten und/oder nitrosylierten α-adrenergen Rezeptorantagonisten nach einem der Ansprüche 1 - 3 und einen pharmazeutisch akzeptablen Träger.

5. Zusammensetzung, umfassend den nitrosierten und/oder nitrosylierten α-adrenergen Rezeptorantagonisten nach einem der Ansprüche 1 - 3 und mindestens eine Verbindung, die Stickstoffmonoxid abgibt, überträgt oder freisetzt, die endogenen Synthesekonzentrationen von Stickstoffmonoxid erhöht oder die endogene Stickstoffmonoxidsynthese stimuliert, die L-Arginin, ein S-Nitrosothiol oder
(i) eine Verbindung, umfassend mindestens eine ON-O-, ON-N- oder ON-C-Gruppe;
(ii) eine Verbindung, umfassend mindestens eine O₂N-O-, O₂N-N-, O₂N-S- oder O₂N-C-Gruppe;
(iii) ein N-Oxo-N-nitrosamin, umfassend eine R₁R₂-N(O-M⁺)-NO-Gruppe, worin R₁ und R₂ jeweils unabhängig ein Polypeptid, eine Aminosäure, ein Zucker, ein modifiziertes oder nicht modifiziertes Oligonucleotid, ein gerader oder verzweigter, gesättigter oder ungesättigter, substituierter oder unsubstituierter, aliphatischer oder aromatischer Kohlenwasserstoff oder eine heterocyclische Verbindung sind; und M⁺ ein Metallkation ist; oder
(iv) ein Thionitrat mit der Struktur R₁₀-S-NO₂, worin R₁₀ ein Polypeptid, eine Aminosäure, ein Zucker, ein modifiziertes oder nicht modifiziertes Oligonucleotid, ein gerader oder verzweigter, gesättigter oder ungesättigter, substituierter oder unsubstituierter, aliphatischer oder aromatischer Kohlenwasserstoff oder eine heterocyclische Verbindung ist, ist.

6. Zusammensetzung nach Anspruch 5, wobei das S-Nitrosothiol S-Nitroso-N-acetylcystein, S-Nitrosocaptopril, S-Nitrosohomocystein, S-Nitrosocystein oder S-Nitrosoglutathion ist.

7. Zusammensetzung nach Anspruch 5, wobei das S-Nitrosothiol
(i) CH₃(C(Rₑ)(R_{f}))ₓSNO;
(ii) HS(C(Rₑ)(R_{f}))ₓSNO;
(iii) ONS(C(Rₑ)(R_{f}))ₓB oder
(iv) H₂N-CH(CO₂H)-(CH₂)ₓ-C(O)NH-CH(CH₂SNO)-C(O)NH-CH₂-CO₂H ist;
worin x = 2 bis 20; Rₑ und R_{f} jeweils unabhängig aus Wasserstoff, Niederalkyl, Cycloalkyl, Aryl, Heteroaryl, Arylalkyl, Alkylamino oder Dialkylamino ausgewählt sind, oder Rₑ und R_{f} zusammen Carbonyl, Cycloalkyl oder verbrücktes Cycloalkyl sind; und B Fluor, Alkoxy, Cyano, Carboxamido, Cycloalkyl, Arylalkoxy, Alkylsulfinyl, Arylthio, Alkylamino, Dialkylamino, Hydroxy, Carbamoyl, N-Alkylcarbamoyl, N,N-Dialkylcarbamoyl, Amino, Hydroxyl, Carboxyl, Wasserstoff, Nitro oder Aryl ist.

8. Verbindung nach Anspruch 5, wobei die Verbindung, die mindestens eine ON-O-, ON-N- oder ON-C-Gruppe umfaßt, ein ON-N-Polypeptid, ein ON-C-Polypeptid, eine ON-N-Aminosäure, eine ON-C-Aminosäure, ein ON-N-Zucker, ein ON-C-Zucker, ein modifiziertes oder nicht modifiziertes ON-N-Oligonucleotid, ein modifiziertes oder nicht modifiziertes ON-C-Oligonucleotid, ein gerader oder verzweigter, gesättigter oder ungesättigter, aliphatischer oder aromatischer ON-O-Kohlenwasserstoff; ein gerader oder verzweigter, gesättigter oder ungesättigter, substituierter oder unsubstituierter, aliphatischer oder aromatischer ON-N-Kohlenwasserstoff, ein gerader oder verzweigter, gesättigter oder ungesättigter, substituierter oder unsubstituierter, aliphatischer oder aromatischer ON-C-Kohlenwasserstoff, eine heterocyclische ON-N-Verbindung oder eine heterocyclische ON-C-Verbindung ist.

9. Verbindung nach Anspruch 5, wobei die Verbindung, die mindestens eine O₂N-O-, O₂N-N-, O₂N-S- oder O₂N-C-Gruppe umfaßt, ein O₂N-O-Polypeptid, eine O₂N-O-Aminosäure, ein O₂N-O-Zucker, ein modifiziertes oder nicht modifiziertes O₂N-O-Oligonucleotid, ein gerader oder verzweigter, gesättigter oder ungesättigter, substituierter oder unsubstituierter, aliphatischer oder aromatischer O₂N-O-Kohlenwasserstoff; eine heterocyclische O₂N-O-Verbindung; ein O₂N-N-Polypeptid, eine O₂N-N-Aminosäure, ein O₂N-N-Zucker, ein modifiziertes oder nicht modifiziertes O₂N-N-Oligonucleotid, ein gerader oder verzweigter, gesättigter oder ungesättigter, substituierter oder unsubstituierter, aliphatischer oder aromatischer O₂N-N-Kohlenwasserstoff; eine heterocyclische O₂N-N-Verbindung; ein O₂N-S-Polypeptid, eine O₂N-S-Aminosäure, ein O₂N-S-Zucker, ein modifiziertes oder nicht modifiziertes O₂N-S-Oligonucleotid, ein gerader oder verzweigter, gesättigter oder ungesättigter, substituierter oder unsubstituierter, aliphatischer oder aromatischer O₂N-S-Kohlenwasserstoff; eine heterocyclische O₂N-S-Verbindung; ein O₂N-C-Polypeptid, eine O₂N-C-Aminosäure, ein O₂N-C-Zucker, ein modifiziertes oder nicht modifiziertes O₂N-C-Oligonucleotid, ein gerader oder verzweigter, gesättigter oder ungesättigter, substituierter oder unsubstituierter, aliphatischer oder aromatischer O₂N-C-Kohlenwasserstoff oder eine heterocyclische O₂N-C-Verbindung ist.

10. Zusammensetzung nach Anspruch 5, wobei die Verbindung, die Stickstoffmonoxid abgibt, überträgt oder freisetzt, ein Nitrat mit der Struktur R₁₀-S-NO₂ ist, worin R₁₀ ein Polypeptid, eine Aminosäure, ein Zucker, ein modifiziertes oder nicht modifiziertes Oligonucleotid, ein gerader oder verzweigter, gesättigter oder ungesättigter, substituierter oder unsubstituierter, aliphatischer oder aromatischer Kohlenwasserstoff oder eine heterocyclische Verbindung ist.

11. Verbindung nach Anspruch 1, die (9S,8R)-5,8-Bis(methoxycarbonyl)-6,7,8,9,10,1 1α,6α,7α-decahydrobenzo(4,3-γ)indolo(2,3-β)chinolizin-9-yl-3-methyl-3-(nitrosothio)butanoat und Säureadditionssalze davon ist.

12. Verbindung nach Anspruch 1, die 4-(2-(Dimethylamino)ethoxy)-2-methyl-5-(1-methylethyl)phenol-3-methyl-3-(nitrosothio)butanoat und Säureadditionssalze davon ist.

13. Zusammensetzung nach Anspruch 5, umfassend einen α-adrenergen Rezeptorantagonisten, ausgewählt aus Yohimbin und Moxisylyt, und eine Verbindung, die Stickstoffmonoxid abgibt, überträgt oder freisetzt, oder die endogenen Synthesekonzentrationen an Stickstoffmonoxid erhöht, die L-Arginin, ein S-Nitrosothiol oder
(i) eine Verbindung, umfassend mindestens eine ON-O-, ON-N- oder ON-C-Gruppe;
(ii) eine Verbindung, umfassend mindestens eine O₂N-O-, O₂N-N-, O₂N-S- oder O₂N-C-Gruppe;
(iii) ein N-Oxo-N-nitrosamin, umfassend eine R₁R₂-N(O-M⁺)-NO-Gruppe, worin R₁ und R₂ jeweils unabhängig ein Polypeptid, eine Aminosäure, ein Zucker, ein modifiziertes oder nicht modifiziertes Oligonucleotid, ein gerader oder verzweigter, gesättigter oder ungesättigter, substituierter oder unsubstituierter, aliphatischer oder aromatischer Kohlenwasserstoff oder eine heterocyclische Verbindung sind; und M⁺ ein Metallkation ist; oder
(iv) ein Thionitrat mit der Struktur R₁₀-S-NO₂, worin R₁₀ ein Polypeptid, eine Aminosäure, ein Zucker, ein modifiziertes oder nicht modifiziertes Oligonucleotid, ein gerader oder verzweigter, gesättigter oder ungesättigter, substituierter oder unsubstituierter, aliphatischer oder aromatischer Kohlenwasserstoff oder eine heterocyclische Verbindung ist, ist.

14. Zusammensetzung nach Anspruch 13, wobei der α-adrenerge Rezeptorantagonist Moxisylyt ist.

15. Zusammensetzung nach Anspruch 13, wobei der α-adrenerge Rezeptorantagonist Yohimbin ist.

16. Zusammensetzung nach Anspruch 13, wobei das S-Nitrosothiol S-Nitroso-N-acetylcystein, S-Nitrosocaptopril, S-Nitrosohomocystein, S-Nitrosocystein oder S-Nitrosoglutathion ist.

17. Zusammensetzung nach Anspruch 13, wobei das S-Nitrosothiol
(i) CH₃(C(Rₑ)(R_{f}))ₓSNO;
(ii) HS(C(Rₑ)(R_{f}))ₓSNO;
(iii) ONS(C(Rₑ)(R_{f}))ₓB oder
(iv) H₂N-CH(CO₂H)-(CH₂)ₓ-C(O)NH-CH(CH₂SNO)-C(O)NH-CH₂-CO₂H ist;
worin x = 2 bis 20; Rₑ und R_{f} jeweils unabhängig aus Wasserstoff, Niederalkyl, Cycloalkyl, Aryl, Heteroaryl, Arylalkyl, Alkylamino oder Dialkylamino ausgewählt sind, oder Rₑ und R_{f} zusammen Carbonyl, Cycloalkyl oder verbrücktes Cycloalkyl sind; und B Fluor, Alkoxy, Cyano, Carboxamido, Cycloalkyl, Arylalkoxy, Alkylsulfinyl, Arylthio, Alkylamino, Dialkylamino, Hydroxy, Carbamoyl, N-Alkylcarbamoyl, N,N-Dialkylcarbamoyl, Amino, Hydroxyl, Carboxyl, Wasserstoff, Nitro oder Aryl ist.

18. Verbindung nach Anspruch 13, wobei die Verbindung, die mindestens eine ON-O-, ON-N- oder ON-C-Gruppe umfaßt, ein ON-N-Polypeptid, ein ON-C-Polypeptid, eine ON-N-Aminosäure, eine ON-C-Aminosäure, ein ON-N-Zucker, ein ON-C-Zucker, ein modifiziertes oder nicht modifiziertes ON-N-Oligonucleotid, ein modifiziertes oder nicht modifiziertes ON-C-Oligonucleotid, ein gerader oder verzweigter, gesättigter oder ungesättigter, aliphatischer oder aromatischer ON-O-Kohlenwasserstoff; ein gerader oder verzweigter, gesättigter oder ungesättigter, substituierter oder unsubstituierter, aliphatischer oder aromatischer ON-N-Kohlenwasserstoff, ein gerader oder verzweigter, gesättigter oder ungesättigter, substituierter oder unsubstituierter, aliphatischer oder aromatischer ON-C-Kohlenwasserstoff, eine heterocyclische ON-N-Verbindung oder eine heterocyclische ON-C-Verbindung ist.

19. Verbindung nach Anspruch 13, wobei die Verbindung, die mindestens eine O₂N-O-, O₂N-N-, O₂N-S- oder O₂N-C-Gruppe umfaßt, ein O₂N-O-Polypeptid, eine O₂N-O-Aminosäure, ein O₂N-O-Zucker, ein modifiziertes oder nicht modifiziertes O₂N-O-Oligonucleotid, ein gerader oder verzweigter, gesättigter oder ungesättigter, substituierter oder unsubstituierter, aliphatischer oder aromatischer O₂N-O-Kohlenwasserstoff; eine heterocyclische O₂N-O-Verbindung; ein O₂N-N-Polypeptid, eine O₂N-N-Aminosäure, ein O₂N-N-Zucker, ein modifiziertes oder nicht modifiziertes O₂N-N-Oligonucleotid, ein gerader oder verzweigter, gesättigter oder ungesättigter, substituierter oder unsubstituierter, aliphatischer oder aromatischer O₂N-N-Kohlenwasserstoff; eine heterocyclische O₂N-N-Verbindung; ein O₂N-S-Polypeptid, eine O₂N-S-Aminosäure, ein O₂N-S-Zucker, ein modifiziertes oder nicht modifiziertes O₂N-S-Oligonucleotid, ein gerader oder verzweigter, gesättigter oder ungesättigter, substituierter oder unsubstituierter, aliphatischer oder aromatischer O₂N-S-Kohlenwasserstoff; eine heterocyclische O₂N-S-Verbindung; ein O₂N-C-Polypeptid, eine O₂N-C-Aminosäure, ein O₂N-C-Zucker, ein modifiziertes oder nicht modifiziertes O₂N-C-Oligonucleotid, ein gerader oder verzweigter, gesättigter oder ungesättigter, substituierter oder unsubstituierter, aliphatischer oder aromatischer O₂N-C-Kohlenwasserstoff oder eine heterocyclische O₂N-C-Verbindung ist.

20. Zusammensetzung nach einem der Ansprüche 13 bis 15, wobei die Verbindung, die Stickstoffmonoxid abgibt, überträgt oder freisetzt oder die endogenen Synthesekonzentrationen an Stickstoffmonoxid erhöht, ein Nitrat mit der Struktur R₁₀-S-NO₂ ist, worin R₁₀ ein Polypeptid, eine Aminosäure, ein Zucker, ein modifiziertes oder nicht modifiziertes Oligonucleotid, ein gerader oder verzweigter, gesättigter oder ungesättigter, substituierter oder unsubstituierter, aliphatischer oder aromatischer Kohlenwasserstoff oder eine heterocyclische Verbindung ist.

21. Zusammensetzung, umfassend Yohimbin und L-Arginin.

22. Zusammensetzung nach einem der Ansprüche 13 bis 21, die ferner einen pharmazeutisch akzeptablen Träger umfaßt.

23. Zusammensetzung nach Anspruch 4 oder Anspruch 21, wobei die Zusammensetzung in oral verabreichbarer Form vorliegt.

24. Zusammensetzung nach Anspruch 23, wobei die oral verabreichbare Form eine feste Dosierform oder eine flüssige Dosierform ist.

25. Zusammensetzung nach Anspruch 24, wobei die feste Dosierform eine Kapsel, eine Tablette, ein Pulver, ein Granulat oder ein Gel ist.

26. Zusammensetzung nach Anspruch 24, wobei die flüssige Dosierform eine Emulsion, eine Lösung, eine Suspension, ein Sirup oder ein Elixier ist.

27. Zusammensetzung nach Anspruch 4 oder Anspruch 22, wobei die Zusammensetzung in einer topisch verabreichbaren Form vorliegt.

28. Kit, umfassend die Zusammensetzung nach Anspruch 5 oder Anspruch 16 bis 21.

29. Kit, umfassend Yohimbin und L-Arginin.

30. Kit nach Anspruch 29, wobei das Yohimbin und L-Arginin separate Komponenten in dem Kit sind.

31. Verwendung einer Zusammensetzung nach einem der Ansprüche 4 oder 16 bis 21 bei der Herstellung eines Medikaments zur Behandlung menschlicher Impotenz bei einem Individuum, das einer solchen bedarf.

32. Verwendung des nitrosierten und/oder nitrosylierten α-adrenergen Rezeptorantagonisten nach Anspruch 1 und einer Verbindung, die Stickstoffmonoxid abgibt, überträgt oder freisetzt, die endogenen Synthesekonzentrationen an Stickstoffmonoxid erhöht oder die endogene Stickstoffmonoxidsynthese stimuliert, wie in Anspruch 5 definiert, bei der Herstellung eines Medikaments zur Behandlung menschlicher Impotenz bei einem Individuum, das einer solchen bedarf.

33. Verwendung nach Anspruch 31 oder Anspruch 32, wobei die menschliche Impotenz männliche Impotenz oder weibliche Impotenz ist.

34. Verwendung von Yohimbin und L-Arginin bei der Herstellung eines Medikaments zur Behandlung menschlicher Impotenz bei einem Individuum, das einer solchen bedarf.

35. Verwendung nach Anspruch 34, wobei das Medikament zur oralen oder topischen Verabreichung vorgesehen ist.

## Revendications

1. Antagoniste des récepteurs α-adrénergiques nitrosé et/ou nitrosylé, lequel antagoniste des récepteurs α-adrénergiques est un composé qui a été nitrosé et/ou nitrosylé, au niveau d'un atome d'oxygène, d'azote ou de soufre, lequel est un composé de formule (III) ou (VIII), le composé de formule (III) étant un composé de formule 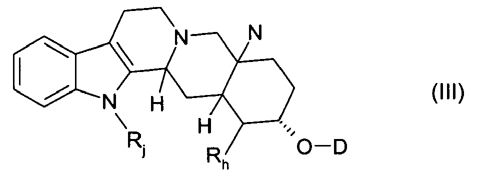 dans laquelle
Rₕ représente un atome d'hydrogène ou un groupe de formule -C(O)-OR_{d} ou -C(O)-X ;
X représente
1) un reste symbolisé par -Y-(C(Rₑ)(R_{f}))ₚ-Gᵢ-(C(Rₑ)(R_{f}))ₚ-T-Q,
2) ou un groupe de formule 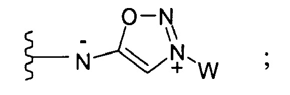
Gᵢ représente une liaison covalente ou un reste symbolisé par -T-C(O)-, -C(O)-T- ou -C(Y-C(O)-Rₘ)- ;
Rₘ représente un groupe hétéroaryle ou un hétérocycle ;
W représente un hétérocycle ou un groupe de formule -NRᵢRᵢ' où Rᵢ
et Rᵢ' représentent chacun, indépendamment, un groupe alkyle inférieur, aryle ou alcényle ;
Rⱼ représente un reste symbolisé par -D ou -C(O)-R_{d} ;
a représente le nombre entier 2 ou 3 ;
D représente :
i) un groupe de formule -NO,
ii) un groupe de formule -NO₂,
iii) un reste symbolisé par -C(R_{d})-O-C(O)-Y-Z-(C(Rₑ)(R_{f}))ₚ-T-Q,
iv) un reste symbolisé par -C(O)-T¹-Z-(C(Rₑ)(R_{f}))ₚ-T²-Q,
v) ou un reste symbolisé par -C(O)-T-(C(R_{y})(R₂))ₚ;
R_{d} représente un atome d'hydrogène ou un groupe alkyle inférieur, cycloalkyle, aryle, alkyl-aryle ou hétéroaryle ;
Y représente un atome d'oxygène ou de soufre ou un groupe de formule NRᵢ où Rᵢ représente un atome d'hydrogène ou un groupe alkyle inférieur ;
Rₑ et R_{f} représentent chacun, indépendamment, un atome d'hydrogène ou un groupe alkyle inférieur, cycloalkyle, aryle, hétéroaryle, aryl-alkyle, amino, alkylamino, amido, alkylamido, dialkylamino ou carboxyle, ou bien Rₑ et R_{f} représentent conjointement un substituant oxo ou un groupe cycloalkyle ou cycloalkyle à pont ;
p représente un nombre entier valant de 1 à 6 ;
T représente un atome d'oxygène, de soufre ou d'azote ;
Z représente une liaison covalente, un groupe alkyle, cycloalkyle, aryle, hétéroaryle ou aryl-alkyle ou un aryl-hétérocycle ;
Q représente un groupe de formule -NO ou -NO₂ ;
T¹ et T² représentent chacun T, indépendamment ;
R_{y} et R_{z} représentent chacun, indépendamment, un reste symbolisé par -T¹-(C(Rₑ)(R_{f}))ₚ-G-(C(Rₑ)(R_{f}))ₚ-T²-Q ;
et G représente une liaison covalente, ou un reste symbolisé par -T-C(O)-, -C(O)-T- ou Y ;
et le composé de formule (VIII) étant un composé de formule 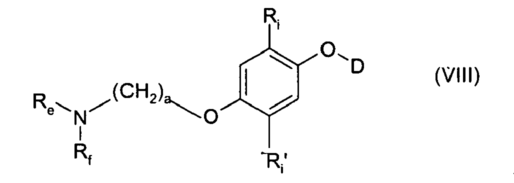 dans laquelle les symboles a, Rᵢ, Rᵢ', Rₑ et R_{f} ont les significations indiquées plus haut.

2. Antagoniste des récepteurs α-adrénergiques nitrosé et/ou nitrosylé, conforme à la revendication 1, lequel antagoniste des récepteurs α-adrénergiques est un dérivé nitrosé et/ou nitrosylé du moxisylyte.

3. Antagoniste des récepteurs α-adrénergiques nitrosé et/ou nitrosylé, conforme à la revendication 1, lequel antagoniste des récepteurs α-adrénergiques est un dérivé nitrosé et/ou nitrosylé de la yohimbine.

4. Composition comprenant un antagoniste des récepteurs α-adrénergiques nitrosé et/ou nitrosylé, conforme à l'une des revendications 1 à 3, et un véhicule pharmacologiquement admissible.

5. Composition comprenant un antagoniste des récepteurs α-adrénergiques nitrosé et/ou nitrosylé, conforme à l'une des revendications 1 à 3, et au moins un composé qui peut donner, transférer ou libérer de l'oxyde nitrique, élever le niveau de synthèse endogène d'oxyde nitrique ou stimuler la synthèse endogène d'oxyde nitrique, lequel composé est dé la L-arginine, un S-nitrosothiol ou
i) un composé comprenant au moins un groupe de formule ON-O-, ON-N- ou ON-C-,
ii) un composé comprenant au moins un groupe de formule O₂N-O-, O₂N-N-, O₂N-S- ou O₂N-C-,
iii) une N-oxo-N-nitrosoamine comprenant un groupe de formule R₁R₂N(O-M⁺)-NO, où R₁ et R₂ représentent chacun, indépendamment, un polypeptide, un acide aminé, un sucre, un oligonucléotide modifié ou non-modifié, un hydrocarbure aliphatique ou aromatique, à chaîne droite ou ramifiée, saturé ou insaturé, avec ou sans substituant(s), ou un composé hétérocyclique, et M⁺ représente un cation métallique,
iv) ou un thionitrate de formule R₁₀-S-NO₂ où R₁₀ représente un polypeptide, un acide aminé, un sucre, un oligonucléotide modifié ou non-modifié, un hydrocarbure aliphatique ou aromatique, à chaîne droite
ou ramifiée, saturé ou insaturé, avec ou sans substituant(s), ou un composé hétérocyclique.

6. Composition conforme à la revendication 5, dans laquelle le S-nitrosothiol est de la S-nitroso-N-acétyl-cystéine, du S-nitroso-captopril, de la S-nitroso-homocystéine, de la S-nitroso-cystéine ou du S-nitroso-glutathion.

7. Composition conforme à la revendication 5, dans laquelle le S-nitrosothiol est un composé de formule
i) H₃C-(C(Rₑ)(R_{f}))ₓ-S-NO,
ii) HS-(C(Rₑ)(R_{f}))ₓ-S-NO,
iii) ON-S-(C(Rₑ)(R_{f}))ₓ-B,
iv) ou H₂N-CH(CO₂H)-(CH₂)ₓ-C(O)NH-CH(CH₂-SNO)-C(O)NH-CO₂H,
où l'indice x vaut de 2 à 20, Rₑ et R_{f} représentent chacun, indépendamment, un atome d'hydrogène ou un groupe alkyle inférieur, cycloalkyle, aryle, hétéroaryle, aryl-alkyle, alkylamino ou dialkylamino, ou bien Rₑ et R_{f} représentent conjointement un substituant oxo ou un groupe cycloalkyle ou cycloalkyle à pont, et B représente un atome d'hydrogène ou de fluor ou un groupe alcoxy, cyano, carboxamido, cycloalkyle, aryl-alcoxy, alkyl-sulfinyle, arylthio, alkylamino, dialkylamino, hydroxy, carbamyle, N-alkyl-carbamyle, N,N-dialkyl-carbamyle, amino, hydroxyle, carboxyle, nitro ou aryle.

8. Composition conforme à la revendication 5, dans laquelle le composé comprenant au moins un groupe de formule ON-O-, ON-N- ou ON-C- est un ON-N-polypeptide, un ON-C-polypeptide, un ON-N-acide aminé, un ON-C-acide aminé, un ON-N-sucre, un ON-C-sucre, un ON-N-oligonucléotide modifié ou non-modifié, un ON-C-oligonucléotide modifié ou non-modifié, un ON-O-hydrocarbure aliphatique ou aromatique, à chaîne droite ou ramifiée, saturé ou insaturé, un ON-N-hydrocarbure aliphatique ou aromatique, à chaîne droite ou ramifiée, saturé ou insaturé, avec ou sans substituant(s), un ON-C-hydrocarbure aliphatique ou aromatique, à chaîne droite ou ramifiée, saturé ou insaturé, avec ou sans substituant(s), un composé ON-N-hétérocyclique ou un composé ON-C-hétérocyclique.

9. Composition conforme à la revendication 5, dans laquelle le composé comprenant au moins un groupe de formule O₂N-O-, O₂N-N-, O₂N-S- ou O₂N-C- est un O₂N-O-polypeptide, un O₂N-O-acide aminé, un O₂N-O-sucre, un O₂N-O-oligonucléotide modifié ou non-modifié, un O₂N- O-hydrocarbure aliphatique ou aromatique, à chaîne droite ou ramifiée, saturé ou insaturé, avec ou sans substituant(s), un composé O₂N-O-hétéro-cyclique, un O₂N-N-polypeptide, un O₂N-N-acide aminé, un O₂N-N-sucre, un O₂N-N-oligonucléotide modifié ou non-modifié, un O₂N-N-hydrocarbure aliphatique ou aromatique, à chaîne droite ou ramifiée, saturé ou insaturé, avec ou sans substituant(s), un composé O₂N-N-hétérocyclique, un O₂N-S- polypeptide, un O₂N-S-acide aminé, un O₂N-S-sucre, un O₂N-S-oligo-nucléotide modifié ou non-modifié, un O₂N-S-hydrocarbure aliphatique ou aromatique, à chaîne droite ou ramifiée, saturé
ou insaturé, avec ou sans substituant(s), un composé O₂N-S-hétérocyclique, un O₂N-C-polypeptide, un O₂N-C-acide aminé, un O₂N-C-sucre, un O₂N-C-oligonucléotide modi-fié ou non-modifié, un O₂N-C-hydrocarbure aliphatique ou aromatique, à chaîne droite ou ramifiée, saturé
ou insaturé, avec ou sans substituant(s), ou un composé O₂N-C-hétérocyclique.

10. Composition conforme à la revendication 5, dans laquelle le composé qui peut donner, transférer ou libérer de l'oxyde nitrique est un nitrate de formule R₁₀-S-NO₂ où R₁₀ représente un polypeptide, un acide aminé, un sucre, un oligonucléotide modifié ou non-modifié, un hydrocarbure aliphatique ou aromatique, à chaîne droite ou ramifiée, saturé ou insaturé, avec ou sans substituant(s), ou un composé hétérocyclique.

11. Composé conforme à la revendication 1, qui est le 3-méthyl-3-(nitrosothio)-butanoate de (9S,8R)-5,8-bis(méthoxycarbonyl)-6,7,8,9,10,11α,6α,7α-décahydrobenzo[4,3-γ]indolo-[2,3-β]quinolizin-9-yle ou l'un de ses sels d'addition d'acide.

12. Composé conforme à la revendication 1, qui est le 3-méthyl-3-(nitrosothio)-butanoate de 4-[(2-diméthylamino)éthoxy]-2-méthyl-5-(1-méthyl-éthyl)phénol ou l'un de ses sels d'addition d'acide.

13. Composition conforme à la revendication 5, comprenant un antagoniste des récepteurs α-adrénergiques, choisi parmi la yohimbine et le moxisylyte, et au moins un composé qui peut donner, transférer ou libérer de l'oxyde nitrique ou élever le niveau de synthèse endogène d'oxyde nitrique, lequel composé est de la L-arginine, un S-nitrosothiol ou
i) un composé comprenant au moins un groupe de formule ON-O-, ON-N- ou ON-C-,
ii) un composé comprenant au moins un groupe de formule O₂N-O-, O₂-N-N-, O₂N-S- ou O₂N-C-,
iii) une N-oxo-N-nitrosoamine comprenant un groupe de formule R₁R₂N(O-M⁺)-NO, où R₁ et R₂ représentent chacun, indépendamment, un polypeptide, un acide aminé, un sucre, un oligonucléotide modifié
ou non-modifié, un hydrocarbure aliphatique ou aromatique, à chaîne droite ou ramifiée, saturé ou insaturé, avec ou sans substituant(s), ou un composé hétérocyclique, et M⁺ représente un cation métallique,
iv) ou un thionitrate de formule R₁₀-S-NO₂ où R₁₀ représente un polypeptide, un acide aminé, un sucre, un oligonucléotide modifié ou non-modifié, un hydrocarbure aliphatique ou aromatique, à chaîne droite
ou ramifiée, saturé ou insaturé, avec ou sans substituant(s), ou un composé hétérocyclique.

14. Composition conforme à la revendication 13, dans laquelle l'antagoniste des récepteurs α-adrénergiques est de la yohimbine.

15. Composition conforme à la revendication 13, dans laquelle l'antagoniste des récepteurs α-adrénergiques est du moxisylyte.

16. Composition conforme à la revendication 13, dans laquelle le S-nitrosothiol est de la S-nitroso-N-acétyl-cystéine, du S-nitroso-captopril, de la S-nitroso-homocystéine, de la S-nitroso-cystéine ou du S-nitroso-glutathion.

17. Composition conforme à la revendication 13, dans laquelle le S-nitrosothiol est un composé de formule
i) H₃C-(C(Rₑ)(R_{f}))ₓ-S-NO,
ii) HS-(C(Rₑ)(R_{f}))ₓ-S-NO,
iii) ON-S-(C(Rₑ)(R_{f}))ₓ-B,
iv) ou H₂N-CH(CO₂H)-(CH₂)ₓ-C(O)NH-CH(CH₂-SNO)-C(O)NH-CO₂H,
où l'indice x vaut de 2 à 20, Rₑ et R_{f} représentent chacun, indépendamment, un atome d'hydrogène ou un groupe alkyle inférieur, cycloalkyle, aryle, hétéroaryle, aryl-alkyle, alkylamino ou dialkylamino, ou bien Rₑ et R_{f} représentent conjointement un substituant oxo ou un groupe cycloalkyle ou cycloalkyle à pont, et B représente un atome d'hydrogène ou de fluor ou un groupe alcoxy, cyano, carboxamido, cycloalkyle, aryl-alcoxy, alkyl-sulfinyle, arylthio, alkylamino, dialkylamino, hydroxy, carbamyle, N-alkyl-carbamyle, N,N-dialkyl-carbamyle, amino, hydroxyle, carboxyle, nitro ou aryle.

18. Composition conforme à la revendication 13, dans laquelle le composé comprenant au moins un groupe de formule ON-O-, ON-N- ou ON-C- est un ON-N-polypeptide, un ON-C-polypeptide, un ON-N-acide aminé, un ON-C-acide aminé, un ON-N-sucre, un ON-C-sucre, un ON-N-oligonucléotide modifié ou non-modifié, un ON-C-oligonucléotide modifié ou non-modifié, un ON-O-hydrocarbure aliphatique ou aromatique, à chaîne droite ou ramifiée, saturé ou insaturé, un ON-N-hydrocarbure aliphatique ou aromatique, à chaîne droite ou ramifiée, saturé ou insaturé, avec ou sans substituant(s), un ON-C-hydrocarbure ali-phatique ou aromatique, à chaîne droite ou ramifiée, saturé ou insaturé, avec ou sans substituant(s), un composé ON-N-hétérocyclique ou un composé ON-C-hétérocyclique.

19. Composition conforme à la revendication 13, dans laquelle le composé comprenant au moins un groupe de formule O₂N-O-, O₂N-N-, O₂N-S- ou O₂N-C- est un O₂N-O-polypeptide, un O₂N-O-acide aminé, un O₂N-O-sucre, un O₂N-O-oligonucléotide modifié ou non-modifié, un O₂N- O-hydrocarbure aliphatique ou aromatique, à chaîne droite ou ramifiée, saturé ou insaturé, avec ou sans substituant(s), un composé O₂N-O-hétéro-cyclique, un O₂N-N-polypeptide, un O₂N-N-acide aminé, un O₂N-N-sucre, un O₂N-N-oligonucléotide modifié ou non-modifié, un O₂N-N-hydrocarbure aliphatique ou aromatique, à chaîne droite ou ramifiée, saturé ou insaturé, avec ou sans substituant(s), un composé O₂N-N-hétérocyclique, un O₂N-S- polypeptide, un O₂N-S-acide aminé, un O₂N-S-sucre, un O₂N-S-oligo-nucléotide modifié ou non-modifié, un O₂N-S-hydrocarbure aliphatique ou aromatique, à chaîne droite ou ramifiée, saturé ou insaturé, avec ou sans substituant(s), un composé O₂N-S-hétérocyclique, un O₂N-C-polypeptide, un O₂N-C-acide aminé, un O₂N-C-sucre, un O₂N-C-oligonucléotide modi-fié ou non-modifié, un O₂N-C-hydrocarbure aliphatique ou aromatique, à chaîne droite ou ramifiée, saturé ou insaturé, avec ou sans substituant(s), ou un composé O₂N-C-hétérocyclique.

20. Composition conforme à l'une des revendications 13 à 15, dans laquelle le composé qui peut donner, transférer ou libérer de l'oxyde nitrique ou élever le niveau de synthèse endogène d'oxyde nitrique est un nitrate de formule R₁₀-S-NO₂ où R₁₀ représente un polypeptide, un acide aminé, un sucre, un oligonucléotide modifié ou non-modifié, un hydrocarbure aliphatique ou aromatique, à chaîne droite ou ramifiée, saturé ou insaturé, avec ou sans substituant(s), ou un composé hétérocyclique.

21. Composition comprenant de la yohimbine et de la L-arginine.

22. Composition conforme à l'une des revendications 13 à 21, qui comprend en outre un véhicule pharmacologiquement admissible.

23. Composition conforme à la revendication 4 ou 22, laquelle composition se présente sous forme administrable par voie orale.

24. Composition conforme à la revendication 23, pour laquelle la forme administrable par voie orale est une forme posologique solide ou une forme posologique liquide.

25. Composition conforme à la revendication 24, pour laquelle la forme posologique solide est une gélule, un comprimé, une poudre, un granulé ou un gel.

26. Composition conforme à la revendication 24, pour laquelle la forme posologique liquide est une émulsion, une solution, une suspension, un sirop ou un élixir.

27. Composition conforme à la revendication 4 ou 22, laquelle composition se présente sous forme administrable par voie topique.

28. Trousse comprenant une composition conforme à l'une des revendications 5 et 16 à 21.

29. Trousse comprenant de la yohimbine et de la L-arginine.

30. Trousse conforme à la revendication 29, dans laquelle la yohimbine et la L-arginine constituent des composants séparés à l'intérieur de cette trousse.

31. Emploi d'une composition conforme à l'une des revendications 4 et 16 à 21 dans la fabrication d'un médicament conçu pour le traitement de l'impuissance humaine chez un individu qui en a besoin.

32. Emploi d'un antagoniste des récepteurs α-adrénergiques nitrosé et/ou nitrosylé, conforme à la revendication 1, et d'un composé qui peut donner, transférer ou libérer de l'oxyde nitrique, élever le niveau de synthèse endogène d'oxyde nitrique ou stimuler la synthèse endogène d'oxyde nitrique, tel que défini dans la revendication 5, dans la fabrication d'un médicament conçu pour le traitement de l'impuissance humaine chez un individu qui en a besoin.

33. Emploi conforme à la revendication 31 ou 32, l'impuissance humaine étant une impuissance masculine ou une impuissance féminine.

34. Emploi de la yohimbine et de la L-arginine dans la fabrication d'un médicament conçu pour le traitement de l'impuissance humaine chez un individu qui en a besoin.

35. Emploi conforme à la revendication 34, le médicament étant conçu pour être administré par voie orale ou topique.
